# EUROPEAN PATENT SPECIFICATION

(11) **EP 1 535 928 B1**
(45) Date of publication and mention of the grant of the patent: **16.07.2008**
(21) Application number: 05003039.4
(22) Date of filing: 22.10.1998
(51) Int. Cl.: C07K 14/22, C12N 15/31, A61K 39/095

(54) **Vaccine compositions comprising Omp85 proteins of Neisseria gonorrhoeae and Neisseria meningitidis**
Vakzine enthaltend Omp85 Proteine von Neisseria gonorrhoeae und Neisseria meningitidis
Vaccin comprenant Omp85 protéines de Neisseria gonorhoeae et Neisseria meningitidis

(43) Date of publication of application: 01.06.2005
(62) Divisional of application: 98953873.1
(73) Proprietor: THE UNIVERSITY OF MONTANA, Missoula, MT 59812 (US)
(72) Inventor: Judd, Ralph C., Florence, Montana 59833 (US); Manning, Scott D., Missoula, Montana 59801 (US)
(74) Representative: Manaton, Ross Timothy

(56) References cited:
- EP-A- 0 474 313
- WO-A-92/03467
- MANNING D S ET AL: "OMP85 PROTEINS OF NEISSERIA GONORRHOEAE AND NEISSERIA MENINGITIDIS ARE SIMILAR TO HAEMOPHILUS INFLUENZAE D-15-AG AND PASTEURELLA MULTOCIDA OMA87" MICROBIAL PATHOGENESIS, ACADEMIC PRESS LIMITED, NEW YORK, NY, US, vol. 25, no. 1, July 1998 (1998-07), pages 11-21, XP000857391 ISSN: 0882-4010 -& MANNING D.S. ET AL.: "Neisseria gonorrhoeae outer membrane protein (omp85) gene, complete cds" EMPRO DATABASE ; ACCESSION NUMBER U81959, 22 February 1997 (1997-02-22), XP002107049 -& MANNING D.S. ET AL.: "Neisseria meningitis outer membrane protein (omp85) gene, complete cds" EMPRO DATABASE ; ACCESSION NUMBER AF021245, 3 October 1997 (1997-10-03), XP002107050

### SEQUENCE LISTING

<110>The University of Montana
<120> Omp85 proteins of Neisseria gonorrhoeae and Neisseria meningitidis, Compositions Containing Same and Methods of Use Thereof
<130> UM/S8C147:DIVEPO
<140>
   <141>
<160> 8
<170> Patentin Ver. 2.0
<210> 1
   <211> 2379
   <212> DNA
   <213> Neisseria gonorrhoeae
<220>
   <221> CDS
   <222> (1)..(2376)
<400> 1
   atg aaa ctg aaa cag att gcc tcc gca ctg atg atg ttg ggc ata tcg   48
   Met Lys Leu Lys Gln Ile Ala Ser Ala Leu Met Met Leu Gly Ile Ser
     1               5                  10                  15
   cct ttg gca ttt gcc gac ttc acc atc caa gac atc cgt gtc gaa ggc   96
   Pro Leu Ala Phe Ala Asp Phe Thr Ile Gln Asp Ile Arg Val Glu Gly
                20                  25                  30
   ttg cag cgt acc gag ccg agc acc gta ttc aac tac ctg ccc gtc aaa   144
   Leu Gln Arg Thr Glu Pro Ser Thr Val Phe Asn Tyr Leu Pro Val Lys
            35                  40                  45
   gtc ggc gac acc tac aac gac aca cac ggc agt gcc atc atc aaa agc   192
   Val Gly Asp Thr Tyr Asn Asp Thr His Gly Ser Ala Ile Ile Lys Ser
        50                  55                  60
   ctg tac gcc acc ggt ttc ttt gac gac gta cga gtc gaa act gcg gac   240
   Leu Tyr Ala Thr Gly Phe Phe Asp Asp Val Arg Val Glu Thr Ala Asp
    65                  70                  75                  80
   ggg ctg ctt ctg ctg acc gtt atc gta tgc cct acc atc ggc tcg ctc     288
   Gly Leu Leu Leu Leu Thr Val Ile Val Cys Pro Thr Ile Gly Ser Leu
                    85                  90                  95
   aac atc acc ggc gcc aaa atg ctg cag aac gac gcc atc aag aaa aac     336
   Asn Ile Thr Gly Ala Lys Met Leu Gln Asn Asp Ala Ile Lys Lys Asn
               100                 105                 110
   ctc gaa tcg ttc ggg ctg gcg cag tcg caa tac ttt aat cag gcg aca     384
   Leu Glu Ser Phe Gly Leu Ala Gln Ser Gln Tyr Phe Asn Gln Ala Thr
           115                 120                 125
   ctc aac cag gca gtc gcc ggc ctg aaa gaa gaa tat ctc ggg cgc ggc     432
   Leu Asn Gln Ala Val Ala Gly Leu Lys Glu Glu Tyr Leu Gly Arg Gly
       130                 135                 140
   aaa ctc aat atc caa atc acg ccc aaa gta acc aaa ctc gcc cgc aac     480
   Lys Leu Asn Ile Gln Ile Thr Pro Lys Val Thr Lys Leu Ala Arg Asn
   145                 150                 155                 160
   cgc gtc gac atc gac atc acg att gac gag ggc aaa tcc gcc aaa atc     528
   Arg Val Asp Ile Asp Ile Thr Ile Asp Glu Gly Lys Ser Ala Lys Ile
                   165                 170                 175
   acc gac atc gaa ttt gaa ggc aac caa gtc tat tcc gac cgc aaa ctg     576
   Thr Asp Ile Glu Phe Glu Gly Asn Gln Val Tyr Ser Asp Arg Lys Leu
               180                 185                 190
   atg cgg cag atg tcg ctg acc gaa ggc ggc att tgg aca tgg ctg aca     624
   Met Arg Gln Met Ser Leu Thr Glu Gly Gly Ile Trp Thr Trp Leu Thr
           195                 200                 205
   cga agc gac cgg ttc gac cgc cag aaa ttc gcc caa gac atg gaa aaa     672
   Arg Ser Asp Arg Phe Asp Arg Gln Lys Phe Ala Gln Asp Met Glu Lys
       210                 215                 220
   gta acc gac ttc tac cag aac aac ggc tac ttc gat ttc cgt atc ctc     720
   Val Thr Asp Phe Tyr Gln Asn Asn Gly Tyr Phe Asp Phe Arg Ile Leu
   225                 230                 235                 240
   gat acc gac atc caa acc aac gaa gac aaa acc agg cag acc atc aaa     768
   Asp Thr Asp Ile Gln Thr Asn Glu Asp Lys Thr Arg Gln Thr Ile Lys
                   245                 250                 255
   atc acc gtc cac gaa ggc gga cgt ttc cgc tgg ggc aaa gtg tcg att     816
   Ile Thr Val His Glu Gly Gly Arg Phe Arg Trp Gly Lys Val Ser Ile
               260                 265                 270
   gaa ggc gac acc aac gaa gtc ccc aag gcc gaa ctg gaa aaa ctg ctg    864
   Glu Gly Asp Thr Asn Glu Val Pro Lys Ala Glu Leu Glu Lys Leu Leu
           275                 280                 285
   acc atg aag ccc ggc aaa tgg tac gaa cgc cag cag atg acc gcc gtt    912
   Thr Met Lys Pro Gly Lys Trp Tyr Glu Arg Gln Gln Met Thr Ala Val
       290                 295                 300
   ttg ggt gag att cag aac cgc atg ggc tcg gca ggc tac gca tac agc    960
   Leu Gly Glu Ile Gln Asn Arg Met Gly Ser Ala Gly Tyr Ala Tyr Ser
   305                 310                 315                 320
   gaa atc agc gta cag ccg ctg ccg aac gcc gga acc aaa acc gtc gat   1008
   Glu Ile Ser Val Gln Pro Leu Pro Asn Ala Gly Thr Lys Thr Val Asp
                   325                 330                 335
   ttc gtc ctg cac atc gaa ccg ggc aga aaa atc tac gtc aac gaa atc   1056
   Phe Val Leu His Ile Glu Pro Gly Arg Lys Ile Tyr Val Asn Glu Ile
               340                 345                 350
   cac atc acc ggc aac aac aaa acc cgc gac gaa gtc gtg cgc cgc gaa   1104
   His Ile Thr Gly Asn Asn Lys Thr Arg Asp Glu Val Val Arg Arg Glu
           355                 360                 365
   ttg cgc caa atg gaa tcc gcg cct tac gac acc tcc aag ctg caa cgc   1152
   Leu Arg Gln Met Glu Ser Ala Pro Tyr Asp Thr Ser Lys Leu Gln Arg
       370                 375                 380
   tcc aaa gag cgc gtc gag ctt ttg ggc tac ttc gac aac gta cag ttt   1200
   Ser Lys Glu Arg Val Glu Leu Leu Gly Tyr Phe Asp Asn Val Gln Phe
   385                 390                 395                 400
   gat gcc gtc ccg ctt gcc ggt acg ccc gac aaa gtc gat ttg aac atg   1248
   Asp Ala Val Pro Leu Ala Gly Thr Pro Asp Lys Val Asp Leu Asn Met
                   405                 410                 415
   agc ctg acc gaa cgt tcc acc ggc tcg ctc gac ttg agc gcg ggc tgg   1296
   Ser Leu Thr Glu Arg Ser Thr Gly Ser Leu Asp Leu Ser Ala Gly Trp
               420                 425                 430
   gtt cag gat acc ggc ttg gtc atg tcc gcc ggc gta tcg cag gac aac   1344
   Val Gln Asp Thr Gly Leu Val Met Ser Ala Gly Val Ser Gln Asp Asn
           435                 440                 445
   ctg ttc ggt acg ggc aag tcg gcc gcc ctg cgc gcc tcg cga agc aaa   1392
   Leu Phe Gly Thr Gly Lys Ser Ala Ala Leu Arg Ala Ser Arg Ser Lys
       450                 455                 460
   acc acg ctc aac ggc tcg ctg tcg ttt acc gac ccg tac ttc acg gca    1440
   Thr Thr Leu Asn Gly Ser Leu Ser Phe Thr Asp Pro Tyr Phe Thr Ala
   465                 470                 475                 480
   gac ggg gtc agc ctg ggc tac gat att tac gga aaa gcc ttc gac ccg    1488
   Asp Gly Val Ser Leu Gly Tyr Asp Ile Tyr Gly Lys Ala Phe Asp Pro
                   485                 490                 495
   cgc aaa gca tcg acc agc gtc aaa caa tat aaa acc acc acc gcc ggc    1536
   Arg Lys Ala Ser Thr Ser Val Lys Gln Tyr Lys Thr Thr Thr Ala Gly
               500                 505                 510
   ggc ggc gta agg atg ggt atc ccc gtt acc gaa tac gac cgc gtc aat    1584
   Gly Gly Val Arg Met Gly Ile Pro Val Thr Glu Tyr Asp Arg Val Asn
           515                 520                 525
   ttc ggg ctg gcg gcg gaa cac ctg acc gtc aac acc tac aac aaa gca    1632
   Phe Gly Leu Ala Ala Glu His Leu Thr Val Asn Thr Tyr Asn Lys Ala
       530                 535                 540
   ccc aaa cgc tat gcc gac ttt atc aaa caa tac ggc aaa acc gac ggc    1680
   Pro Lys Arg Tyr Ala Asp Phe Ile Lys Gln Tyr Gly Lys Thr Asp Gly
   545                 550                 555                 560
   gca gac ggc agc ttc aaa ggc ctg ctg tac aaa ggc act gtc ggc tgg    1728
   Ala Asp Gly Ser Phe Lys Gly Leu Leu Tyr Lys Gly Thr Val Gly Trp
                   565                 570                 575
   ggg cgc aac aag acc gac agc gcc tta tgg ccg acg cgc ggc tac ctg    1776
   Gly Arg Asn Lys Thr Asp Ser Ala Leu Trp Pro Thr Arg Gly Tyr Leu
               580                 585                 590
   acc ggc gta aat gcc gaa atc gcc ctg ccc ggc agc aaa ctg caa tac    1824
   Thr Gly Val Asn Ala Glu Ile Ala Leu Pro Gly Ser Lys Leu Gln Tyr
           595                 600                 605
   tac tcc gcc acc cac aac caa acc tgg ttc ttc ccc tta agc aaa acc    1872
   Tyr Ser Ala Thr His Asn Gln Thr Trp Phe Phe Pro Leu Ser Lys Thr
       610                 615                 620
   ttc acg ctg atg ctc ggc ggc gaa gtc ggc att gcg ggc ggc tac ggc    1920
   Phe Thr Leu Met Leu Gly Gly Glu Val Gly Ile Ala Gly Gly Tyr Gly
   625                 630                 635                 640
   aga acc aaa gaa atc ccc ttc ttt gaa aac ttc tac ggc ggc ggc ctg    1968
   Arg Thr Lys Glu Ile Pro Phe Phe Glu Asn Phe Tyr Gly Gly Gly Leu
                   645                 650                 655
   ggt tcg gtg cgc ggc tac gaa agc ggc acg ctc ggc ccg aaa gtg tat     2016
   Gly Ser Val Arg Gly Tyr Glu Ser Gly Thr Leu Gly Pro Lys Val Tyr
               660                 665                 670
   gac gaa tac ggc gaa aaa atc agc tac ggc ggc aac aaa aaa gcc aac     2064
   Asp Glu Tyr Gly Glu Lys Ile Ser Tyr Gly Gly Asn Lys Lys Ala Asn
           675                 680                 685
   gtc tcc gcc gag ctg ctt ttc ccg atg ccc ggt gcg aaa gac gca cgc     2112
   Val Ser Ala Glu Leu Leu Phe Pro Met Pro Gly Ala Lys Asp Ala Arg
       690                 695                 700
   acc gtc cgc ctg agc ctg ttt gcc gac gca ggc agc gtg tgg gac ggc     2160
   Thr Val Arg Leu Ser Leu Phe Ala Asp Ala Gly Ser Val Trp Asp Gly
   705                 710                 715                 720
   aga acc tat acc gcc gcc gaa aac ggt aac aac aaa tcg gtt tac tcg     2208
   Arg Thr Tyr Thr Ala Ala Glu Asn Gly Asn Asn Lys Ser Val Tyr Ser
                   725                 730                 735
   gaa aac gcg cat aaa tcc acc ttt acc aac gaa ttg cgc tat tcc gcc     2256
   Glu Asn Ala His Lys Ser Thr Phe Thr Asn Glu Leu Arg Tyr Ser Ala
               740                 745                 750
   ggc ggc gcg gtt acc tgg ctc tcg cct ttg ggc ccg atg aaa ttc atc     2304
   Gly Gly Ala Val Thr Trp Leu Ser Pro Leu Gly Pro Met Lys Phe Ile
           755                 760                 765
   tac gcc tac ccg ctg aag aaa aaa ccg gaa gac gaa atc caa cgc ttc     2352
   Tyr Ala Tyr Pro Leu Lys Lys Lys Pro Glu Asp Glu Ile Gln Arg Phe
       770                 775                 780
   caa ttc cag ctc ggc acg acg ttc taa                                 2379
   Gln Phe Gln Leu Gly Thr Thr Phe
   785                 790
<210> 2
   <211> 792
   <212> PRT
   <213> Neisseria gonorrhoeae
<400> 2
   Met Lys Leu Lys Gln Ile Ala Ser Ala Leu Met Met Leu Gly Ile Ser
   1               5                   10                  15
   Pro Leu Ala Phe Ala Asp Phe Thr Ile Gln Asp Ile Arg Val Glu Gly
   20 25 30
   Leu Gln Arg Thr Glu Pro Ser Thr Val Phe Asn Tyr Leu Pro Val Lys
   35 40 45
   Val Gly Asp Thr Tyr Asn Asp Thr His Gly Ser Ala Ile Ile Lys Ser
   50 55 60
   Leu Tyr Ala Thr Gly Phe Phe Asp Asp Val Arg Val Glu Thr Ala Asp
   65 70 75 80
   Gly Leu Leu Leu Leu Thr Val Ile Val Cys Pro Thr Ile Gly Ser Leu
   85 90 95
   Asn Ile Thr Gly Ala Lys Met Leu Gln Asn Asp Ala Ile Lys Lys Asn
   100 105 110
   Leu Glu Ser Phe Gly Leu Ala Gln Ser Gln Tyr Phe Asn Gln Ala Thr
   115 120 125
   Leu Asn Gln Ala Val Ala Gly Leu Lys Glu Glu Tyr Leu Gly Arg Gly
   130 135 140
   Lys Leu Asn Ile Gln Ile Thr Pro Lys Val Thr Lys Leu Ala Arg Asn
   145 150 155 160
   Arg Val Asp Ile Asp Ile Thr Ile Asp Glu Gly Lys Ser Ala Lys Ile
   165 170 175
   Thr Asp Ile Glu Phe Glu Gly Asn Gln Val Tyr Ser Asp Arg Lys Leu
   180 185 190
   Met Arg Gln Met Ser Leu Thr Glu Gly Gly Ile Trp Thr Trp Leu Thr
   195 200 205
   Arg Ser Asp Arg Phe Asp Arg Gln Lys Phe Ala Gln Asp Met Glu Lys
   210 215 220
   Val Thr Asp Phe Tyr Gln Asn Asn Gly Tyr Phe Asp Phe Arg Ile Leu
   225 230 235 240
   Asp Thr Asp Ile Gln Thr Asn Glu Asp Lys Thr Arg Gln Thr Ile Lys
   245 250 255
   Ile Thr Val His Glu Gly Gly Arg Phe Arg Trp Gly Lys Val Ser Ile
   260 265 270
   Glu Gly Asp Thr Asn Glu Val Pro Lys Ala Glu Leu Glu Lys Leu Leu
   275 280 285
   Thr Met Lys Pro Gly Lys Trp Tyr Glu Arg Gln Gln Met Thr Ala Val
   290 295 300
   Leu Gly Glu Ile Gln Asn Arg Met Gly Ser Ala Gly Tyr Ala Tyr Ser
   305 310 315 320
   Glu Ile Ser Val Gln Pro Leu Pro Asn Ala Gly Thr Lys Thr Val Asp
   325 330 335
   Phe Val Leu His Ile Glu Pro Gly Arg Lys Ile Tyr Val Asn Glu Ile
   340 345 350
   His Ile Thr Gly Asn Asn Lys Thr Arg Asp Glu Val Val Arg Arg Glu
   355 360 365
   Leu Arg Gln Met Glu Ser Ala Pro Tyr Asp Thr Ser Lys Leu Gln Arg
   370 375 380
   Ser Lys Glu Arg Val Glu Leu Leu Gly Tyr Phe Asp Asn Val Gln Phe
   385 390 395 400
   Asp Ala Val Pro Leu Ala Gly Thr Pro Asp Lys Val Asp Leu Asn Met
   405 410 415
   Ser Leu Thr Glu Arg Ser Thr Gly Ser Leu Asp Leu Ser Ala Gly Trp
   420 425 430
   Val Gln Asp Thr Gly Leu Val Met Ser Ala Gly Val Ser Gln Asp Asn
   435 440 445
   Leu Phe Gly Thr Gly Lys Ser Ala Ala Leu Arg Ala Ser Arg Ser Lys
   450 455 460
   Thr Thr Leu Asn Gly Ser Leu Ser Phe Thr Asp Pro Tyr Phe Thr Ala
   465 470 475 480
   Asp Gly Val Ser Leu Gly Tyr Asp Ile Tyr Gly Lys Ala Phe Asp Pro
   485 490 495
   Arg Lys Ala Ser Thr Ser Val Lys Gln Tyr Lys Thr Thr Thr Ala Gly
   500 505 510
   Gly Gly Val Arg Met Gly Ile Pro Val Thr Glu Tyr Asp Arg Val Asn
   515 520 525
   Phe Gly Leu Ala Ala Glu His Leu Thr Val Asn Thr Tyr Asn Lys Ala
   530 535 540
   Pro Lys Arg Tyr Ala Asp Phe Ile Lys Gln Tyr Gly Lys Thr Asp Gly
   545 550 555 560
   Ala Asp Gly Ser Phe Lys Gly Leu Leu Tyr Lys Gly Thr Val Gly Trp
   565 570 575
   Gly Arg Asn Lys Thr Asp Ser Ala Leu Trp Pro Thr Arg Gly Tyr Leu
   580 585 590
   Thr Gly Val Asn Ala Glu Ile Ala Leu Pro Gly Ser Lys Leu Gln Tyr
   595 600 605
   Tyr Ser Ala Thr His Asn Gln Thr Trp Phe Phe Pro Leu Ser Lys Thr
   610 615 620
   Phe Thr Leu Met Leu Gly Gly Glu Val Gly Ile Ala Gly Gly Tyr Gly
   625 630 635 640
   Arg Thr Lys Glu Ile Pro Phe Phe Glu Asn Phe Tyr Gly Gly Gly Leu
   645 650 655
   Gly Ser Val Arg Gly Tyr Glu Ser Gly Thr Leu Gly Pro Lys Val Tyr
   660 665 670
   Asp Glu Tyr Gly Glu Lys Ile Ser Tyr Gly Gly Asn Lys Lys Ala Asn
   675 680 685
   Val Ser Ala Glu Leu Leu Phe Pro Met Pro Gly Ala Lys Asp Ala Arg
   690 695 700
   Thr Val Arg Leu Ser Leu Phe Ala Asp Ala Gly Ser Val Trp Asp Gly
   705 710 715 720
   Arg Thr Tyr Thr Ala Ala Glu Asn Gly Asn Asn Lys Ser Val Tyr Ser
   725 730 735
   Glu Asn Ala His Lys Ser Thr Phe Thr Asn Glu Leu Arg Tyr Ser Ala
   740 745 750
   Gly Gly Ala Val Thr Trp Leu Ser Pro Leu Gly Pro Met Lys Phe Ile
   755 760 765
   Tyr Ala Tyr Pro Leu Lys Lys Lys Pro Glu Asp Glu Ile Gln Arg Phe
   770 775 780
   Gln Phe Gln Leu Gly Thr Thr Phe
   785 790
<210> 3
   <211> 2394
   <212> DNA
   <213> Neisseria meningitidis
<220>
   <221> CDS
   <222> (1)..(2391)
<400> 3
   atg aaa ctg aaa cag att gct tcc gca ctg atg atg ttg ggc ata tcg    48
   Met Lys Leu Lys Gln Ile Ala Ser Ala Leu Met Met Leu Gly Ile Ser
     1               5                  10                  15
   cct ttg gca ttt gcc gac ttc acc atc caa gac atc cgt gtc gaa ggc    96
   Pro Leu Ala Phe Ala Asp Phe Thr Ile Gln Asp Ile Arg Val Glu Gly
                20                  25                  30
   ttg cag cgt acc gag ccg agc acc gta ttc aac tac ctg ccc gtc aaa   144
   Leu Gln Arg Thr Glu Pro Ser Thr Val Phe Asn Tyr Leu Pro Val Lys
            35                  40                  45
   gtc ggc gat acc tac aac gac aca cac ggc agt gcc atc atc aaa agc   192
   Val Gly Asp Thr Tyr Asn Asp Thr His Gly Ser Ala Ile Ile Lys Ser
        50                  55                  60
   ctg tac gcc acc ggt ttc ttt gac gac gta cgc gtc gaa act gcg gac   240
   Leu Tyr Ala Thr Gly Phe Phe Asp Asp Val Arg Val Glu Thr Ala Asp
    65                  70                  75                  80
   ggg cag ctc ctg ctg acc gtt atc gaa cgc ccc acc atc ggc tcg ctc   288
   Gly Gln Leu Leu Leu Thr Val Ile Glu Arg Pro Thr Ile Gly Ser Leu
                    85                  90                  95
   aac atc acc ggc gca aaa atg ctg caa aac gac gcc att aag aaa aac   336
   Asn Ile Thr Gly Ala Lys Met Leu Gln Asn Asp Ala Ile Lys Lys Asn
               100                 105                 110
   ctc gaa tcg ttc ggg ctg gcg cag tcg caa tac ttt aat cag gcg aca   384
   Leu Glu Ser Phe Gly Leu Ala Gln Ser Gln Tyr Phe Asn Gln Ala Thr
           115                 120                 125
   ctc aat cag gca gtc gcc ggc ctg aaa gaa gaa tac ctc ggg cgc ggc   432
   Leu Asn Gln Ala Val Ala Gly Leu Lys Glu Glu Tyr Leu Gly Arg Gly
       130                 135                 140
   aaa ctc aat atc caa atc acg ccc aaa gta acc aaa ctc gcc cgc aac     480
   Lys Leu Asn Ile Gln Ile Thr Pro Lys Val Thr Lys Leu Ala Arg Asn
   145                 150                 155                 160
   cgc gtc gac atc gac atc acg att gac gag ggc aaa tcc gcc aaa atc     528
   Arg Val Asp Ile Asp Ile Thr Ile Asp Glu Gly Lys Ser Ala Lys Ile
                   165                 170                 175
   acc gac atc gaa ttt gaa ggc aac caa gtc tat tcc gac cgc aaa ctg     576
   Thr Asp Ile Glu Phe Glu Gly Asn Gln Val Tyr Ser Asp Arg Lys Leu
               180                 185                 190
   atg cgg cag atg tcg ctg acc gaa ggc ggc att tgg aca tgg ctg aca     624
   Met Arg Gln Met Ser Leu Thr Glu Gly Gly Ile Trp Thr Trp Leu Thr
           195                 200                 205
   cga agc aac caa ttc aac gag cag aaa ttt gcc caa gac atg gaa aaa     672
   Arg Ser Asn Gln Phe Asn Glu Gln Lys Phe Ala Gln Asp Met Glu Lys
       210                 215                 220
   gta acc gac ttc tac cag aac aac ggc tac ttc gat ttc cgt atc ctc     720
   Val Thr Asp Phe Tyr Gln Asn Asn Gly Tyr Phe Asp Phe Arg Ile Leu
   225                 230                 235                 240
   gat acc gac atc caa acc aac gaa gac aaa acc aag cag acc atc aaa     768
   Asp Thr Asp Ile Gln Thr Asn Glu Asp Lys Thr Lys Gln Thr Ile Lys
                   245                 250                 255
   atc acc gtc cac gaa ggc gga cgt ttc cgt tgg ggc aaa gtc tcc atc     816
   Ile Thr Val His Glu Gly Gly Arg Phe Arg Trp Gly Lys Val Ser Ile
               260                 265                 270
   gaa ggc gac acc aac gaa gtc ccc aaa gcc gaa ctg gaa aaa ctg ctg     864
   Glu Gly Asp Thr Asn Glu Val Pro Lys Ala Glu Leu Glu Lys Leu Leu
           275                 280                 285
   acc atg aag ccc ggc aaa tgg tac gaa cgc cag cag atg acc gcc gtt     912
   Thr Met Lys Pro Gly Lys Trp Tyr Glu Arg Gln Gln Met Thr Ala Val
       290                 295                 300
   ttg ggt gag att cag aac cgc atg ggc tcg gca ggc tac gca tac agc     960
   Leu Gly Glu Ile Gln Asn Arg Met Gly Ser Ala Gly Tyr Ala Tyr Ser
   305                 310                 315                 320
   gaa atc agc gta cag ccg ctg cca aac gcc gaa acc aaa acc gtc gat     1008
   Glu Ile Ser Val Gln Pro Leu Pro Asn Ala Glu Thr Lys Thr Val Asp
                   325                 330                 335
   ttc gtc ctg cac atc gaa ccg ggc cgg aaa atc tac gtc aac gaa atc     1056
   Phe Val Leu His Ile Glu Pro Gly Arg Lys Ile Tyr Val Asn Glu Ile
               340                 345                 350
   cac atc acc ggc aac aac aaa acc cgc gac gaa gtc gtg cgc cgc gaa     1104
   His Ile Thr Gly Asn Asn Lys Thr Arg Asp Glu Val Val Arg Arg Glu
           355                 360                 365
   ttg cgc caa atg gaa tcc gcg cct tac gac acc tcc aag ctg caa cgc     1152
   Leu Arg Gln Met Glu Ser Ala Pro Tyr Asp Thr Ser Lys Leu Gln Arg
       370                 375                 380
   tcc aaa gag cgc gtc gag ctt ttg ggc tac ttc gac aac gta cag ttt     1200
   Ser Lys Glu Arg Val Glu Leu Leu Gly Tyr Phe Asp Asn Val Gln Phe
   385                 390                 395                 400
   gat gcc gtc ccg ctt gcc ggc aca ccc gac aaa gtc gat ttg aac atg     1248
   Asp Ala Val Pro Leu Ala Gly Thr Pro Asp Lys Val Asp Leu Asn Met
                   405                 410                 415
   agc ctg acc gaa cgt tcc acc ggc tcg ctc gac ttg agc gcg ggc tgg     1296
   Ser Leu Thr Glu Arg Ser Thr Gly Ser Leu Asp Leu Ser Ala Gly Trp
               420                 425                 430
   gta cag gat acc ggc ctg gtc atg tcc gca ggc gtt tcc caa gac aac     1344
   Val Gln Asp Thr Gly Leu Val Met Ser Ala Gly Val Ser Gln Asp Asn
           435                 440                 445
   ctg ttc ggt acg ggc aag tcg gcc gcc ctg cgc gcc tca cga agc aaa     1392
   Leu Phe Gly Thr Gly Lys Ser Ala Ala Leu Arg Ala Ser Arg Ser Lys
       450                 455                 460
   acc acg ctc aac ggc tcg ctg tcg ttt acc gac ccg tac ttc acg gca     1440
   Thr Thr Leu Asn Gly Ser Leu Ser Phe Thr Asp Pro Tyr Phe Thr Ala
   465                 470                 475                 480
   gac ggg gtc agc ctg ggc tac gat gtt tac gga aaa gcc ttc gac ccg     1488
   Asp Gly Val Ser Leu Gly Tyr Asp Val Tyr Gly Lys Ala Phe Asp Pro
                   485                 490                 495
   cgc aaa gca tcg acc agc atc aaa caa tat aaa acc acc acg gca ggc     1536
   Arg Lys Ala Ser Thr Ser Ile Lys Gln Tyr Lys Thr Thr Thr Ala Gly
               500                 505                 510
   gca ggc atc cgc atg agc gtg cct gtt acc gaa tac gac cgc gtg aat     1584
   Ala Gly Ile Arg Met Ser Val Pro Val Thr Glu Tyr Asp Arg Val Asn
           515                 520                 525
   ttc ggt ttg gtg gca gaa cac ctg acc gtc aac acc tac aac aaa gcg    1632
   Phe Gly Leu Val Ala Glu His Leu Thr Val Asn Thr Tyr Asn Lys Ala
       530                 535                 540
   ccc aaa cac tat gcc gac ttt atc aag aaa tac ggc aaa acc gac ggc    1680
   Pro Lys His Tyr Ala Asp Phe Ile Lys Lys Tyr Gly Lys Thr Asp Gly
   545                 550                 555                 560
   aca gac ggc agc ttc aaa ggc tgg ctg tac aaa ggt acc gtc ggc tgg    1728
   Thr Asp Gly Ser Phe Lys Gly Trp Leu Tyr Lys Gly Thr Val Gly Trp
                   565                 570                 575
   ggg cgc aac aaa acc gac agc gcg tta tgg ccg acg cgc ggc tac ctg    1776
   Gly Arg Asn Lys Thr Asp Ser Ala Leu Trp Pro Thr Arg Gly Tyr Leu
               580                 585                 590
   acg ggc gtg aac gcc gaa atc gcc ctg ccc ggc agc aaa ctg caa tac    1824
   Thr Gly Val Asn Ala Glu Ile Ala Leu Pro Gly Ser Lys Leu Gln Tyr
           595                 600                 605
   tac tcc gcc acc cac aac caa acc tgg ttc ttc ccc tta agc aaa acc    1872
   Tyr Ser Ala Thr His Asn Gln Thr Trp Phe Phe Pro Leu Ser Lys Thr
       610                 615                 620
   ttc acg ctg atg ctc ggc ggc gaa gtc ggc att gcg ggc ggc tac ggc    1920
   Phe Thr Leu Met Leu Gly Gly Glu Val Gly Ile Ala Gly Gly Tyr Gly
   625                 630                 635                 640
   aga acc aaa gaa atc ccc ttc ttt gaa aac ttc tac ggc ggc ggc ctg    1968
   Arg Thr Lys Glu Ile Pro Phe Phe Glu Asn Phe Tyr Gly Gly Gly Leu
                   645                 650                 655
   ggt tcg gtg cgc gga tac gaa agc ggc acg ctc ggt ccg aaa gtg tat    2016
   Gly Ser Val Arg Gly Tyr Glu Ser Gly Thr Leu Gly Pro Lys Val Tyr
               660                 665                 670
   gac gaa tac ggc gaa aaa atc agc tac ggc ggc aac aaa aaa gcc aac    2064
   Asp Glu Tyr Gly Glu Lys Ile Ser Tyr Gly Gly Asn Lys Lys Ala Asn
           675                 680                 685
   gtc tcc gcc gag ctg ctc ttc ccg atg cct ggc gcg aaa gac gcg cgc    2112
   Val Ser Ala Glu Leu Leu Phe Pro Met Pro Gly Ala Lys Asp Ala Arg
       690                 695                 700
   acc gtc cgc ctg agc ctg ttt gcc gac gca ggc agc gtg tgg gac ggc    2160
   Thr Val Arg Leu Ser Leu Phe Ala Asp Ala Gly Ser Val Trp Asp Gly
   705                 710                 715                 720
   aaa acc tac gac gac aac agc agt tcc gcg acc ggc ggc agg gtt caa    2208
   Lys Thr Tyr Asp Asp Asn Ser Ser Ser Ala Thr Gly Gly Arg Val Gln
                   725                 730                 735
   aac att tac ggc gcc ggc aat acc cat aaa tcc acc ttt acc aac gaa    2256
   Asn Ile Tyr Gly Ala Gly Asn Thr His Lys Ser Thr Phe Thr Asn Glu
               740                 745                 750
   ttg cgc tat tcc gcc ggc ggc gcg gtt acc tgg ctc tcg cct tta ggc    2304
   Leu Arg Tyr Ser Ala Gly Gly Ala Val Thr Trp Leu Ser Pro Leu Gly
           755                 760                 765
   ccg atg aaa ttc agg tac gcc tac ccg ctg aag aaa aaa ccg gaa gac    2352
   Pro Met Lys Phe Arg Tyr Ala Tyr Pro Leu Lys Lys Lys Pro Glu Asp
       770                 775                 780
   gaa atc caa cgc ttc caa ttc caa ctc ggc acg acg ttc taa            2394
   Glu Ile Gln Arg Phe Gln Phe Gln Leu Gly Thr Thr Phe
   785                 790                 795
<210> 4
   <211> 797
   <212> PRT
   <213> Neisseria meningitidis
<400> 4
   Met Lys Leu Lys Gln Ile Ala Ser Ala Leu Met Met Leu Gly Ile Ser
   1               5                   10                  15
   Pro Leu Ala Phe Ala Asp Phe Thr Ile Gln Asp Ile Arg Val Glu Gly
               20                  25                  30
   Leu Gln Arg Thr Glu Pro Ser Thr Val Phe Asn Tyr Leu Pro Val Lys
           35                  40                  45
   Val Gly Asp Thr Tyr Asn Asp Thr His Gly Ser Ala Ile Ile Lys Ser
       50                  55                  60
   Leu Tyr Ala Thr Gly Phe Phe Asp Asp Val Arg Val Glu Thr Ala Asp
   65                  70                  75                  80
   Gly Gln Leu Leu Leu Thr Val Ile Glu Arg Pro Thr Ile Gly Ser Leu
                   85                  90                  95
   Asn Ile Thr Gly Ala Lys Met Leu Gln Asn Asp Ala Ile Lys Lys Asn
               100                 105                 110
   Leu Glu Ser Phe Gly Leu Ala Gln Ser Gln Tyr Phe Asn Gln Ala Thr
   115 120 125
   Leu Asn Gln Ala Val Ala Gly Leu Lys Glu Glu Tyr Leu Gly Arg Gly
   130 135 140
   Lys Leu Asn Ile Gln Ile Thr Pro Lys Val Thr Lys Leu Ala Arg Asn
   145 150 155 160
   Arg Val Asp Ile Asp Ile Thr Ile Asp Glu Gly Lys Ser Ala Lys Ile
   165 170 175
   Thr Asp Ile Glu Phe Glu Gly Asn Gln Val Tyr Ser Asp Arg Lys Leu
   180 185 190
   Met Arg Gln Met Ser Leu Thr Glu Gly Gly Ile Trp Thr Trp Leu Thr
   195 200 205
   Arg Ser Asn Gln Phe Asn Glu Gln Lys Phe Ala Gln Asp Met Glu Lys
   210 215 220
   Val Thr Asp Phe Tyr Gln Asn Asn Gly Tyr Phe Asp Phe Arg Ile Leu
   225 230 235 240
   Asp Thr Asp Ile Gln Thr Asn Glu Asp Lys Thr Lys Gln Thr Ile Lys
   245 250 255
   Ile Thr Val His Glu Gly Gly Arg Phe Arg Trp Gly Lys Val Ser Ile
   260 265 270
   Glu Gly Asp Thr Asn Glu Val Pro Lys Ala Glu Leu Glu Lys Leu Leu
   275 280 285
   Thr Met Lys Pro Gly Lys Trp Tyr Glu Arg Gln Gln Met Thr Ala Val
   290 295 300
   Leu Gly Glu Ile Gln Asn Arg Met Gly Ser Ala Gly Tyr Ala Tyr Ser
   305 310 315 320
   Glu Ile Ser Val Gln Pro Leu Pro Asn Ala Glu Thr Lys Thr Val Asp
   325 330 335
   Phe Val Leu His Ile Glu Pro Gly Arg Lys Ile Tyr Val Asn Glu Ile
   340 345 350
   His Ile Thr Gly Asn Asn Lys Thr Arg Asp Glu Val Val Arg Arg Glu
   355 360 365
   Leu Arg Gln Met Glu Ser Ala Pro Tyr Asp Thr Ser Lys Leu Gln Arg
   370 375 380
   Ser Lys Glu Arg Val Glu Leu Leu Gly Tyr Phe Asp Asn Val Gln Phe
   385 390 395 400
   Asp Ala Val Pro Leu Ala Gly Thr Pro Asp Lys Val Asp Leu Asn Met
   405 410 415
   Ser Leu Thr Glu Arg Ser Thr Gly Ser Leu Asp Leu Ser Ala Gly Trp
   420 425 430
   Val Gln Asp Thr Gly Leu Val Met Ser Ala Gly Val Ser Gln Asp Asn
   435 440 445
   Leu Phe Gly Thr Gly Lys Ser Ala Ala Leu Arg Ala Ser Arg Ser Lys
   450 455 460
   Thr Thr Leu Asn Gly Ser Leu Ser Phe Thr Asp Pro Tyr Phe Thr Ala
   465 470 475 480
   Asp Gly Val Ser Leu Gly Tyr Asp Val Tyr Gly Lys Ala Phe Asp Pro
   485 490 495
   Arg Lys Ala Ser Thr Ser Ile Lys Gln Tyr Lys Thr Thr Thr Ala Gly
   500 505 510
   Ala Gly Ile Arg Met Ser Val Pro Val Thr Glu Tyr Asp Arg Val Asn
   515 520 525
   Phe Gly Leu Val Ala Glu His Leu Thr Val Asn Thr Tyr Asn Lys Ala
   530 535 540
   Pro Lys His Tyr Ala Asp Phe Ile Lys Lys Tyr Gly Lys Thr Asp Gly
   545 550 555 560
   Thr Asp Gly Ser Phe Lys Gly Trp Leu Tyr Lys Gly Thr Val Gly Trp
   565 570 575
   Gly Arg Asn Lys Thr Asp Ser Ala Leu Trp Pro Thr Arg Gly Tyr Leu
   580 585 590
   Thr Gly Val Asn Ala Glu Ile Ala Leu Pro Gly Ser Lys Leu Gln Tyr
   595 600 605
   Tyr Ser Ala Thr His Asn Gln Thr Trp Phe Phe Pro Leu Ser Lys Thr
   610 615 620
   Phe Thr Leu Met Leu Gly Gly Glu Val Gly Ile Ala Gly Gly Tyr Gly
   625 630 635 640
   Arg Thr Lys Glu Ile Pro Phe Phe Glu Asn Phe Tyr Gly Gly Gly Leu
   645 650 655
   Gly Ser Val Arg Gly Tyr Glu Ser Gly Thr Leu Gly Pro Lys Val Tyr
   660 665 670
   Asp Glu Tyr Gly Glu Lys Ile Ser Tyr Gly Gly Asn Lys Lys Ala Asn
   675 680 685
   Val Ser Ala Glu Leu Leu Phe Pro Met Pro Gly Ala Lys Asp Ala Arg
   690 695 700
   Thr Val Arg Leu Ser Leu Phe Ala Asp Ala Gly Ser Val Trp Asp Gly
   705 710 715 720
   Lys Thr Tyr Asp Asp Asn Ser Ser Ser Ala Thr Gly Gly Arg Val Gln
   725 730 735
   Asn Ile Tyr Gly Ala Gly Asn Thr His Lys Ser Thr Phe Thr Asn Glu
   740 745 750
   Leu Arg Tyr Ser Ala Gly Gly Ala Val Thr Trp Leu Ser Pro Leu Gly
   755 760 765
   Pro Met Lys Phe Arg Tyr Ala Tyr Pro Leu Lys Lys Lys Pro Glu Asp
   770 775 780
   Glu Ile Gln Arg Phe Gln Phe Gln Leu Gly Thr Thr Phe
   785 790 795
<210> 5
   <211> 23
   <212> DNA
   <213> PCR primer
<400> 5
   cggaattcat gaaactgaaa cag 23
<210> 6
   <211> 18
   <212> DNA
   <213> PCR primer
<400> 6
   ttgcagtttt tgcaattc 18
<210> 7
   <211> 59
   <212> DNA
   <213> Neisseria gonorrhoeae
<400> 7
   tagattttac gtttcggaat gcagtctgaa accgcattcc gcaccacaag gaacttacg 59
<210> 8
   <211> 67
   <212> DNA
   <213> Neisseria gonorrhoeae
<400> 8
   cccgcaaatg ccgtctgaag cccttgagac ggcatttcgc ggcaacatcc gaaggagttt 60
   taccatg                                                           67

## Description

This invention has been supported by National Institutes of Health Grant Nos. AI21235 and AI37777. The United States Government has an interest in this invention.

### Field of the Invention

This disclosure relates generally to the cloning and identification of novel outer membrane proteins of several strains of *Neisseria,* and more specifically to proteins useful in the prevention, therapy and/or diagnosis of infection and diseases in mammals caused by these strains.

### Background of the Invention

The pathogenic *Neisseriae* cause several important non-symptomatic infection and symptomatic disease in humans. *Neisseria gonorrhoeae* is the agent of non-symptomatic gonococcal infection or symptomatic disease, i.e., gonorrhea. *Neisseria meningitidis* is the agent of a rapidly progressive spinal meningitis, which may also have a non-symptomatic infective stage. The surface of such pathogens provide crucial interfaces for interactions between the pathogen and the host. Many bacterial virulence factors are outer membrane proteins, and surface exposed proteins are the primary targets recognized and attacked by the host's immune system. Thus, the role of outer membrane proteins is of particular importance in understanding the pathogenesis of these organisms. The most abundant and immunodominant outer membrane proteins of the pathogenic *Neisseriae* have been studied extensively [Sparling P. F. et al, Clin. Invest., 89: 1699-1705 (1992)]. For example, it is known that the immunodominant components of the gonococcal surface are antigenically variant, suggesting that this organism is capable of adapting to varying host environments while avoiding host immune responses. Although the major gonococcal surface proteins have been extensively studied, little is known about less abundant proteins and their contribution to pathogenesis.

Two-dimensional electrophoresis (IEF and SDS-PAGE) of labeled, e.g., radioiodinated or biotinylated, gonococcal surface proteins suggested that numerous (>20) of the less abundant gonococcal outer membrane proteins remained uncharacterized (unpublished observations). Among these might be proteins which play an important role in infection.

For example, surface-exposed outer membrane proteins of other microorganisms, e.g., *Haemophilus influenzae* D15 surface antigen (D-15-Ag) and the *Pasteurella multocida* Oma87 have been found to be useful in eliciting antibodies that were protective against infectious challenge in animal models. The Omp85-!ike D-15-Ag was conserved in both non-typeable and typeable strains of *H*. *influenzae* and was recognized by convalescent patient sera; afnnity-punfied anti-D-15-Ag serum was protective in the rat pup model [Thomas, W. R., et al, Infect. Immun.,58:1909-1913 (1990); Flack, F. S. et al, Gene, 156:97-99(1995)]. *H. influenzae* serotypes a-f, nontypeable *H. influenzae* and *Haemophilus parainfluenzae* all expressed proteins similar to the D-15-Ag, as demonstrated by immunoblot analysis. Antibodies to recombinant D-15-Ag protected against *H. influenzae* type b and type a bacteremia in the infant rat model [Loosmore, S. M. et al, Infect. Immun., 65:3701-3707 (1997)].

Like *H. influenzae* D-15-Ag, the Oma87 of *P. multocida* was highly conserved among strams and was recognized by protective antibody; it was present in all 16 serotypes of *P. multocida* and was recognized by convalescent animal sera. Antibodies raised to recombinant Oma87 were protective against homologous challenge in the mouse model [Ruffolo, C. G. et al., Infect. Immun., 64:3161-3167 (1996)]. Despite the several publications describing the immunological properties of D-15-Ag and Oma87, the function of these proteins remains unknown.

Manning et al., Microbial Pathogenesis 25:11-21 (1998) discloses that Omp 85 proteins of Neisseria gonorrhoeae and Nisseria meningiditis are similar to Haemophilus influenzae D-15-Ag and Pasteurella multocida Oma87.

There remains a need in the art for the development of proteins from Neisseriae which are useful in research, diagnosis and treatment of the infections, especially non-symptomatic infections, and the diseases caused by these pathogens.

### Summary of the Invention

According to a first aspect of the invention, there is provided a vaccine composition for the protection of a subject against Neisserial disease comprising, in a pharmaceutically acceptable carrier, an effective amount of a polypeptide selected from the group consisting of
(a) the protein of SEQ ID NO: 2;
(b) the protein of SEQ ID NO: 4;
(c) a peptide fragment of SEQ ID NO: 2 or 4 consisting of eight or more consecutive amino acids thereof;
(d) a protein or peptide sharing at least 90% identity with any of the sequences of a-(b) and
(e) a sequence of any of (a) - (d) fused to a second polypeptide or protein.
The composition may comprises more than one protein or peptide of any of (a)-(e). The composition may further comprise at least one other antigen or fragment thereof from a heterologous or homologous pathogenic bacterial species. The other antigen may be fused to one of (a)-(c). The polypeptide may lack the signal sequence spanning amino acids 1-21 of SEQ ID NO: 2 or 4.

According to a second aspect of the invention, there is provided a method of manufacturing a composition as described above, comprising the steps of isolating a recombinant polypeptide comprising at least eight consecutive amino acids from the amino acid sequence of SEQ ID NO: 2 or 4, which polypeptide induces antibodies that recognize SEQ ID NO: 2 or 4, and formulating said polypeptide in an amount effective to induce said antibodies in a mammalian subject, with a pharmaceutically acceptable carrier.

According to a third aspect of the invention, there is provided a vaccine composition for the protection of a subject against Neisserial disease comprising, in a suitable nucleic acid delivery vehicle, an effective amount of a nucleic acid sequence selected from the group consisting of:
(a) SEQ ID NO: 1;
(b) SEQ ID NO: 3;
(c) a fragment of SEQ ID NO: I or 3 consisting of 15 or more consecutive nucleic acids thereof;
(d) a sequence encoding a polypeptide sharing at least 90% identity with either SEQ ID NO:2 or SEQ ID NO:4; and
(e) a sequence of any of (a)-(d) further encoding a second polypeptide or protein.

The composition may comprise more than one nucleic acid sequence of any of (a) - (e).

The composition may further comprise at least one other nucleic acid sequence encoding an antigen or fragment thereof from a heterologous or homologous pathogenic bacterial species. The other antigen may be fused to one of (a) - (c)

According to a fourth aspect of the invention there is provided a vaccine composition for the protection of a subject against Neisserial disease comprising an isolated antibody that binds to a protein or peptide selected from the group consisting of:
(a) the protein of SEQ ID NO: 2;
(b) the protein of SEQ ID NO: 4;
(c) a peptide fragment of SEQ ID NO: 2 or 4 consisting of eight or more consecutive amino acids thereof; and
(d) a protein or peptide sharing at least 90% identity with any of the sequences of (a)-(b).

The composition may comprise a single isolated antibody or multiple antibodies.

According to a fifth aspect of the invention, there is provided the use of a composition according to the invention in the manufacture of a medicament for the protection of a subject against Neisserial disease.

### Brief Description of the Drawings

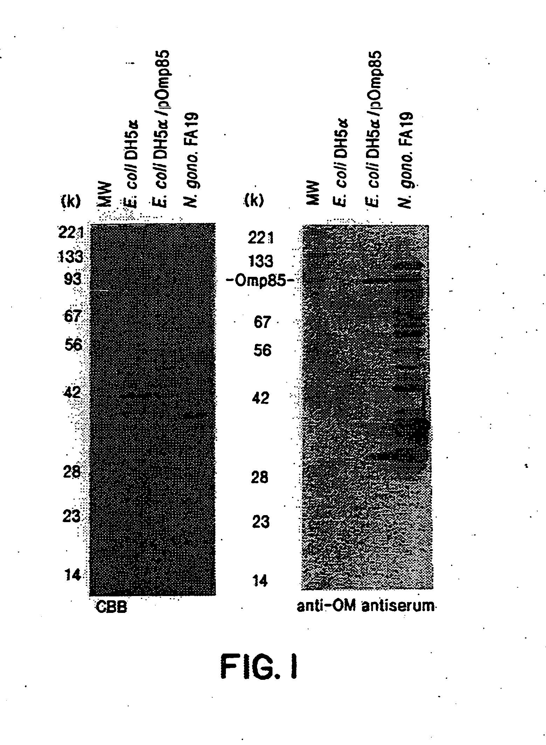

Fig. 1 is a photograph of a sodium dodecyl sulfate polyacrylamide electrophoretic gel (SDS-PAGE) illustrating the identification of recombinant Omp85 produced by *E. coli* DH5α/pOmp85, as described in Example 2. Bacterial cell lysates were separated by SDS-PAGE, stained with Coomassie Brilliant blue (CBB) or transferred to membranes and probed with anti-GC-OM serum. From left to right are *E. coli* DH5α, *E. coli* DH5α/pOmp85, and *N. gonorrhoeae* strain FA19. The location of Omp85 is indicated. Prestained molecular mass markers (MW), were indicated in kilodaltons (kDa).

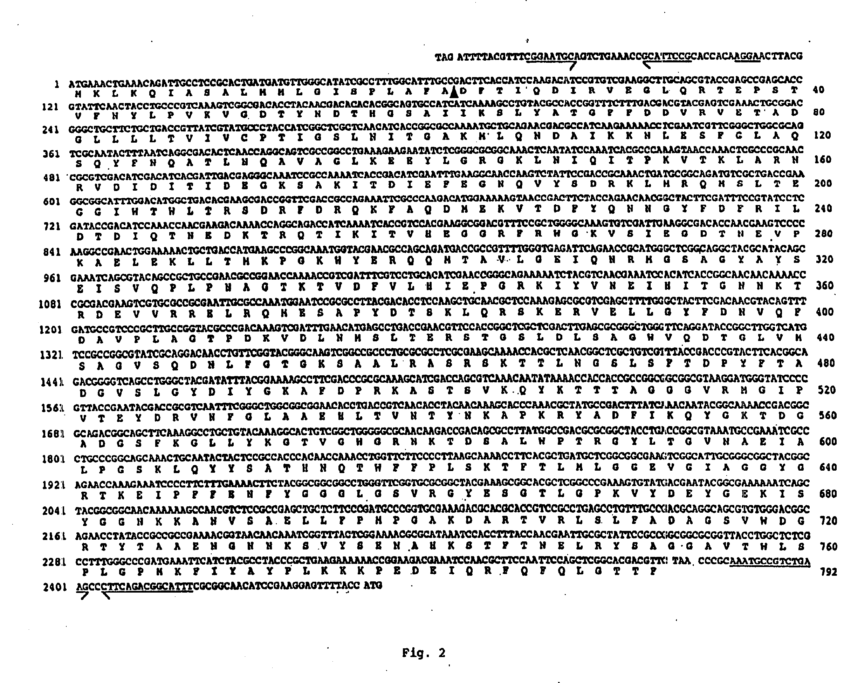

Fig. 2 illustrates the DNA sequence encoding an open reading frame of the *N. gonorrhoeae omp85* [SEQ ID NO: 1] and the corresponding deduced amino acid sequence of Omp85 (Genbank accession #U81959) [SEQ ID NO: 2], which is preceded by an untranslated 5' sequence [SEQ ID NO: 7], and followed by an untranslated 3' sequence [SEQ ID NO: 8]. The nucleotide sequence begins with the termination codon of a preceding open reading frame (ORF) that is similar to that of the *H. influenzae* hypothetical protein HI0918 and ends with the initiation codon of a downstream gene similar to *omp*H of *S. typhimurium* [Kosk P. et al, J. Biol. Chem., 264: 18973-18980 (1989)]. The nucleotides of the *omp85* open reading frame are numbered on the left. A ribosome binding site (underlined) precedes the initiation codon of the *omp85* ORF. The *omp85* ORF was preceded and followed by rho-independent transcriptional termination sequences (indicated by lines with arrows). The Omp85 precursor polypeptide was composed of 792 amino acids. The amino acid sequence is numbered on the right. A putative signal peptide cleavage site was identified (indicated by arrowhead) [Von Heijne, G., Nucl. Acids Res., 14:4683-4690 (1986)]. Cleavage at this site would produce a mature protein having a predicted molecular weight of 85,842 Da.

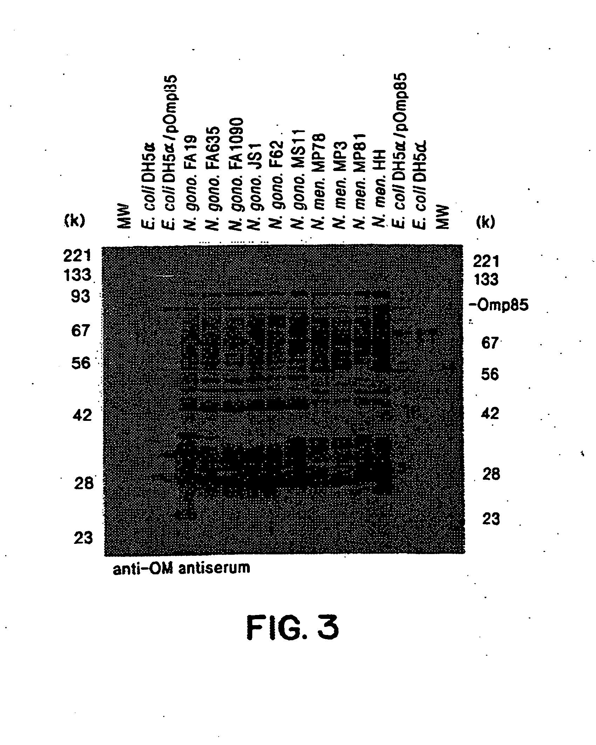

Fig. 3 is a photograph of a Western blot that illustrates the identification of Omp85 in *N*. *gonorrhoeae* strains FA19, FA635, FA1090, JS1, F62 and MS11LosA *and N. meningitidis* strains MP78, MP3, MP81 and HH by Western blot analysis with anti-GC-OM serum. *E. coli* DH5α and *E. coli* DHSα/pOmp85 were used as negative and positive controls. Prestained molecular mass markers (MW) were indicated in kDa.

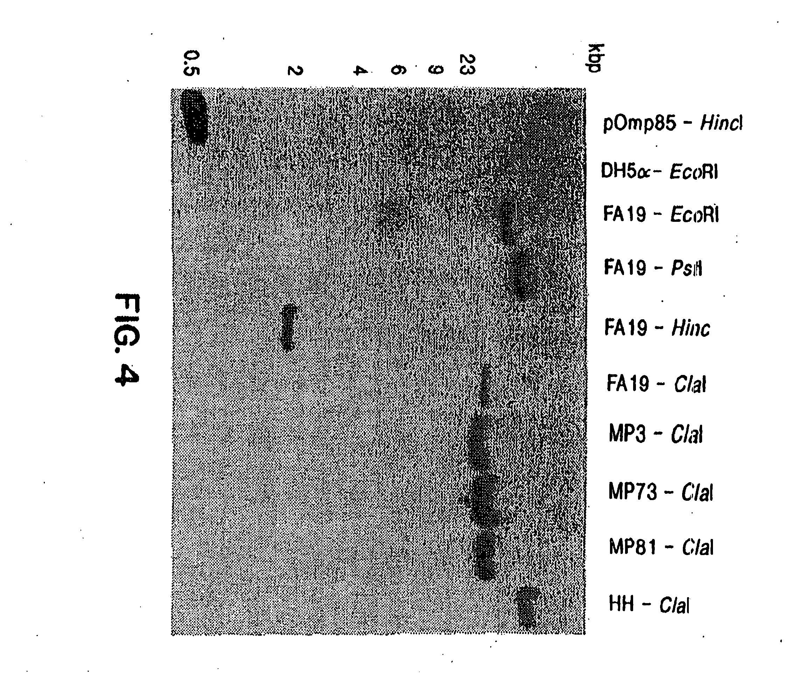

Fig. 4 is a photograph of a Southern blot that illustrates the identification of *omp85* in genomic DNA from *N. gonorrhoeae* FA19 and *N. meningitidis* strains MP3, MP73, MP81 and HH digested with restriction endonucleases (*Hinc*II, *Eco*RI*, Pst*I, *Cla*I) and probed with a 688 bp fragment of gonococcal *omp85.* This fragment wits used as a positive control in the first lane. Molecular weight markers are indicated on the left in kilobase pairs. *E. coli* DH5α was used as a negative control.

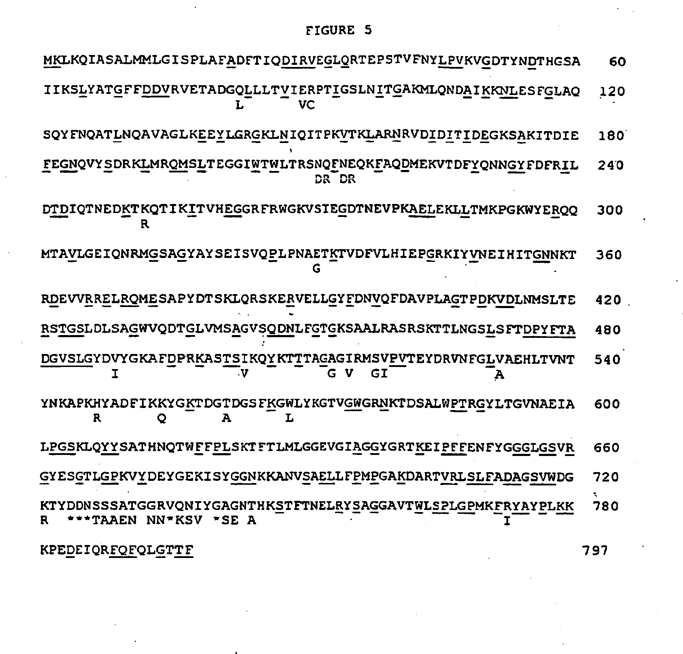

Fig. 5 illustrates the amino acid sequence of *N. meningitidis* Omp85 (Genbank accession #AF021045) [SEQ ID NO: 4] compared to that of *N. gonorrhoeae* Omp85 [SEQ ID NO: 2]. On the top line is the *N. meningitidis* Omp85 amino acid sequence. Below it are the amino acids that are different in the *N. gonorrhoeae* Omp85, Amino acids that are absent in the gonococcal Omp85 are indicated by stars. Amino acids that are identical in the Omp85 homologs of *N*. *mertingitidis, N. gonorrhoeae, H. influenzae* (D-15-Ag) and *P. multocida* (Oma87) are underlined. The amino acids of the meningococcal Omp85 are numbered on the right.

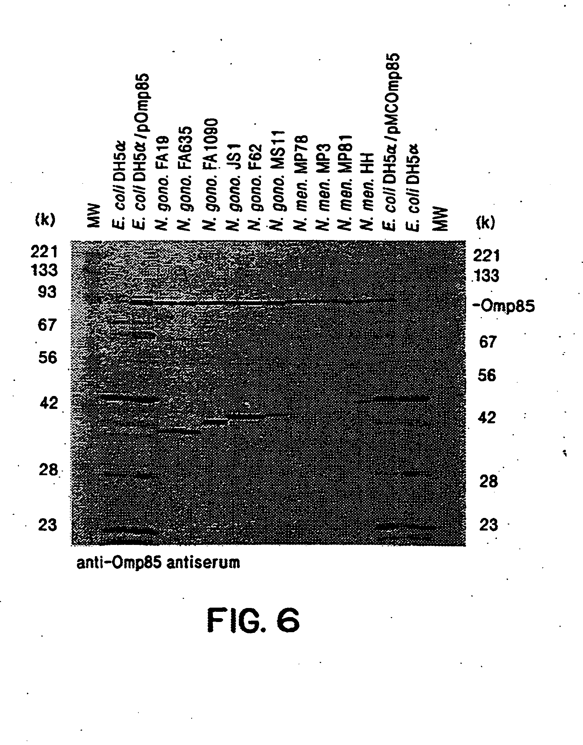

Fig. 6 is a photograph of a Western blot that illustrates the identification of Omp85 in *N. gonorrhoeae* strains FA19, FA635, FA1090, JS1, F62 and MS11LosA and *N. meningitidis* strains MP78, MP3, MP81 and HH by Western blot analysis with anti-Omp85, as described in Example 7. *E. coli* DH5α, *E*. *coli* DHSα/pOmp85 and *E*. *coli* DH5α/pMCOmp85 were used as negative and positive controls. Prestained molecular mass markers (MW) were indicated in kDa.

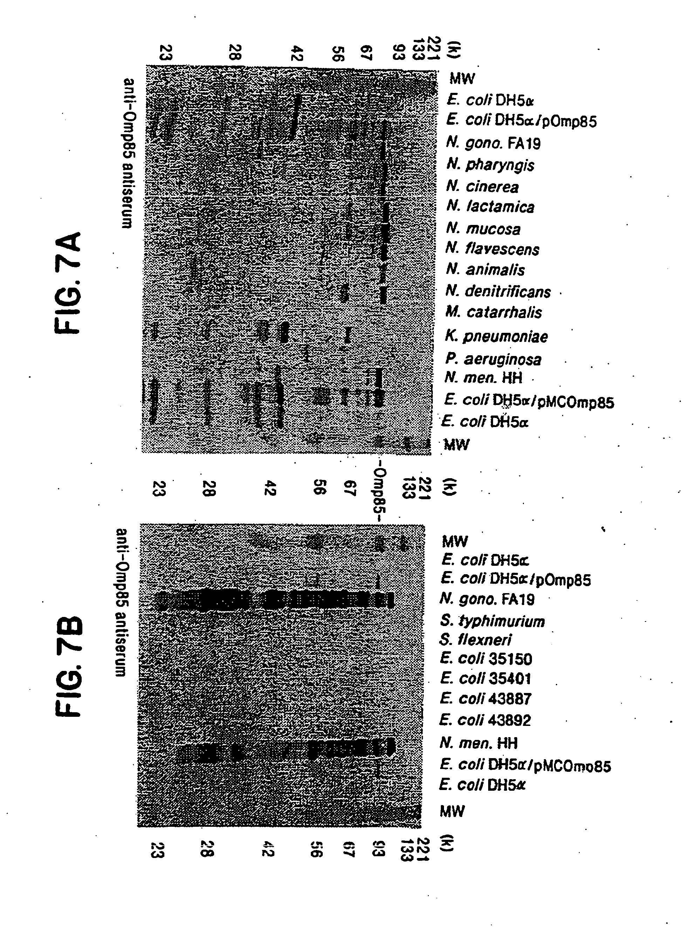

Fig. 7A is a photograph of a Western blot that illustrates the distribution of Omp85 in pathogenic and commensal *Neisseriae* (relationship areas A, B, C, and D) and related Gram negative bacteria: *N. gonorrhoeae* FA 19 (A), *Neisseria pharyngis* (A), *Neisseria cinerea* (A), *Neisseria lactamica* (B), *Neisseria mucosae* (B), *Neisseria flavescens* (C), *Neisseria animalis* (C), *Neisseria denitrificans* (C), *Moraxella catarrhalis* (*D*), *Klebsiella pneumoniae, Pseudomonas aeruginosa,* and *N*. *meningitidis* HH (A), as described in Example 7. *E. coli* DH5α and *E*. *coli* DH5α/pOmp85, were used as negative and positive controls. Prestained molecular mass markers (MW) were indicated in kDa.
Fig. 7B is a photograph of a Western blot that illustrates the distribution of Omp85 in *N. gonorrhoeae* FA19, *Salmonella typhimnrium, Shigella flexneri, E. coli* strains 35150 (enterohemorrhagic - EHEC). 35401 (enterotoxigenic = ETEC), 43887 (enteropathogenic - EPEC), 43892 (enteroinvasive - EIEC) and *N. meningitidis,* as described in Example 7. *E. coli* DH5α and *E. coli* DH5α/pOmp85 were used as negative and positive controls. Prestained molecular mass markers (MW) were indicated in kDa.

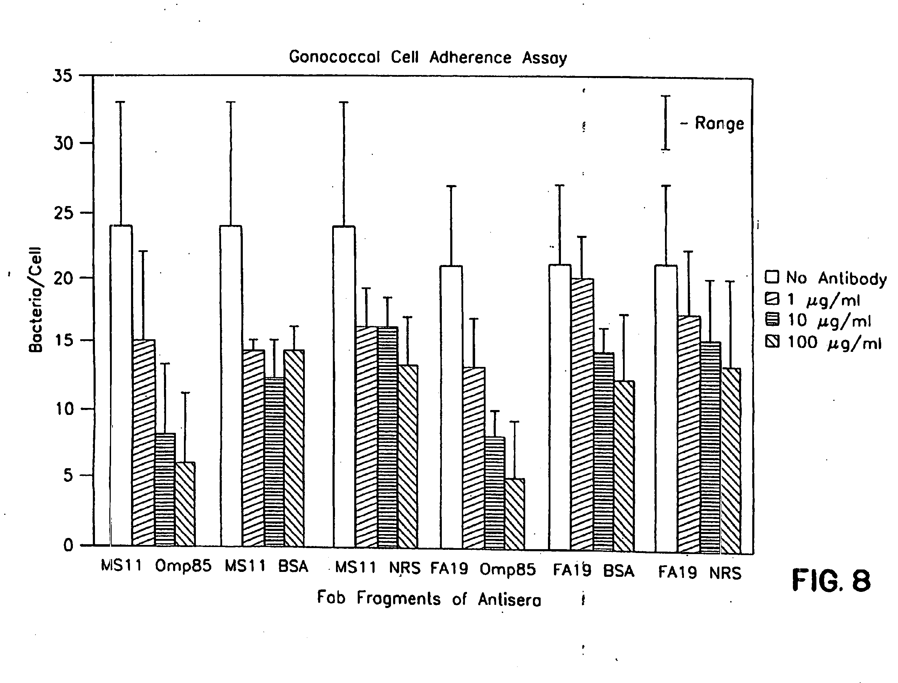

Fig. 8 is a bar graph showing the results of a gonococcal cell adherence assay performed with no antibody (black bars), and with Fab fragments prepared from antisera to: Ms11 Omp85, MS11 hyperimmune sera to bovine serum albumin (MS11BSA), MS11 hyperimmune serum to normal rabbit serum (MS11NRS), FA19 Omp85, FA19BSA and FA19NRS at concentrations of 1, 10 and 100 µg/ml (see key). The assay was performed as described in Example 8.

### Detailed Description of the Invention

The present invention provides vaccine compositions relating to outer surface membrane proteins, referred to as Omp85, from *N. gonorrhoeae* and *N. meningitidis.* These antigens, fragments thereof, antibodies developed thereto, the nucleic acid sequences encoding same, and the use of such antigens, antibodies and nucleic acid sequences provide vaccine compositions and their use for the treatment or prevention of gonococcal and meningococcal infections, particularly non-symptomatic infections, and diseases.

### I. The Omp85 Antigens of Use in the Invention

To identify the previously uncharacterized *N. gonorrhoeae* outer membrane proteins, an *N. gonorrhoeae* genomic library was screened with an antiserum raised against purified isolated gonococcal outer membranes. The gonococcal gene, *omp85,* was identified that encodes a 792 amino acid outer membrane protein, Omp85, of *N. gonorrhoeae* having an apparent molecular weight of 85kDa and characterized by the amino acid sequence of Fig. 2 and SEQ ID NO: 2. Omp85 has a typical signal peptide and a carboxyl-terminal phenylalanine characteristic of outer membrane proteins. Southern analysis demonstrated that the *omp85* gene was present as a single copy in *N. gonorrhoeae* and *N. meningitidis.* PCR amplification was used to obtain a clone of the *N. meningitidis omp85* homolog. The genes encoding the *N. gonorrhoeae* and *N. meningitidis* Omp85 proteins, have been cloned and sequenced. The *omp85* gene and its product in both *N. gonorrhoeae* and *N. meningitidis* are characterized in Figs. 2 and 5 below [SEQ ID NOS: 1-4].

The nucleic acid sequences encoding the Omp85 proteins and the structures of the proteins themselves are described below. Where in this specification, protein and/or DNA sequences are defined by their percent homologies or identities to identified sequences, the algorithms used to calculate the percent identities or percent similarities include the following: the Smith-Waterman algorithm [J. F. Collins et al, 1988, Comput. Appl. Biosci., 4:67-72; J. F. Collins et al, Molecular Sequence Comparison and Alignment, (M. J: Bishop et al, eds.) In Practical Approach Series: Nucleic Acid and Protein Sequence Analysis XVIII, IRL Press: Oxford, England, UK (1987) pp.417], and the BLAST and FASTA programs [E. G. Shpaer et al, 1996, Genomics, 38:179-191], including the BLAST2 program [S. D. Altschul et al, J. Mol. Biol., 215:403-407 (1990)].

### A. Nucleic Acid Sequences

The present invention uses bacterial nucleic acid sequences encoding *omp85* sequences of *N. gonorrhoeae* and *N. meningitidis.* The nucleic acid sequences are isolated from cellular materials with which they are naturally associated. The DNA sequence of the *N. gonorrhoeae omp85* [SEQ ID NO: 1] and the corresponding deduced amino acid sequence of Omp85 (Genbank accession #U81959) [SEQ ID NO: 2] were obtained as described in the Example 2 and in Fig. 2. The nucleotide sequence begins with the termination codon of a preceding ORF that is similar to that of the *H. influenzae* hypothetical protein HI0918 and ends with the initiation codon of a downstream gene similar to *omp*H of *S*. *typhimurium* [Kosk P. et al, J. Biol. Chem., 264: 18973-18980 (1989)]. A ribosome binding site precedes the initiation codon of the *omp85* ORF. The *omp85* ORF was preceded and followed by rho-independent transcriptional termination sequences. The Omp85 precursor polypeptide was composed of 792 amino acids. A putative signal peptide cleavage site was identified (indicated by arrowhead) [Von Heijne, G., Nucl Acids Res., 14:4683-4690 (1986)]. Cleavage at this site produces a mature protein having a predicted molecular weight of 85,842 Da.

The DNA sequence of the *N. meningitidis omp85* [SEQ ID NO: 3] and the corresponding deduced amino acid sequence of Omp85 (Genbank accession #AF021045) [SEQ ID NO: 4] were obtained as described in Example 3. Fig. 5 shows the comparison between the sequences of the two Omp85 proteins, as well as their similarities to the Omp85 homologs of *N. meningitidis, N. gonorrhoeae, H. influerrzae* (D-15-Ag) and *P. multocida* (Oma87).

In addition to the full-length nucleic acid sequences encoding the Omp85 proteins provided herein, the specification also includes fragments of these *omp85* genes. Preferably, such fragments are characterized by encoding a biologically active portion of Omp85, e.g., an epitope. Alternatively, other non-epitopic fragments may be useful as probes in diagnostic or research use. Generally, these oligonucleotide fragments are at least 10, or at least 15 consecutive nucleotides in length. However, oligonucleotide fragments of varying sizes may be selected as desired. Such fragments may be used for such purposes as performing polymerase chain reaction (PCR), e.g., on a biopsied tissue sample. For example, useful fragments of *omp85* DNA and corresponding sequences comprise sequences occurring between nucleotides 2161 through 2208 of SEQ ID NO: 1 and nucleotides 2161 and 2218 of SEQ ID NO: 3. Other useful fragments may be readily obtained by one of skill in the art by resort to conventional DNA sequencing techniques applied to the sequences disclosed herein.

The DNA sequences of SEQ ID NOS: I and 3 permit one of skill in the art to readily obtain the corresponding anti-sense strands of these DNA sequences. Further, using known techniques, one of skill in the art can readily obtain additional genomic and cDNA sequences which flank the illustrated DNA sequences or the corresponding RNA sequences, as desired. Similarly the availability of SEQ ID NOS: 1 and 3 permits one of skill in the art to obtain other species Omp85 analogs, and fragments thereof, by use of the nucleic acid sequences as probes in a conventional technique, e.g., polymerase chain reaction. Allelic variants of these sequences within a species (i.e., sequences containing some individual nucleotide differences from a more commonly occurring sequence within a species, but which nevertheless encode the same protein or a protein with the same function) such as other variants of Omp85 [SEQ ID NOS: 2 and 4] may also be readily obtained given the knowledge of these nucleic acid sequences.

The present invention may further involve nucleic acid sequences for use in the invention capable of hybridizing under stringent conditions [see, J. Sambrook et al, Molecular Cloning: A Laboratory Manual, 2d ed., Cold Spring Harbor Laboratory (1989)] to the sequences of SEQ ID NOS: 1 and 3, their anti-sense strands, or biologically active fragments thereof. An example of a highly stringent hybridization condition is hybridization at 2XSSC at 65°C, followed by a washing in 0.1XSSC at 65°C for an hour. Alternatively, an exemplary highly stringent hybridization condition is in 50% formamide, 4XSSC at 42°C. Moderately high stringency conditions may also prove useful, e.g., hybridization in 4XSSC at 55°C, followed by washing in 0.1XSSC at 37°C for an hour. An alternative exemplary moderately high stringency hybridization condition is in 50% formamide, 4XSSC at 30°C.

The nucleic acid sequences may be modified. Utilizing the sequence data of SEQ ID NOS: 1 and 3, it is within the skill of the art to obtain other synthetically or recombinantly-prepared polynucleotide sequences, or modified polynucleotide sequences, encoding the full-length proteins or useful fragments. For example, one of skill may employ preferred or "preference" codons for expression of the sequence in selected host cells; thus SEQ ID NOS: 1 and 3 may be modified to contain different nucleotide triplets which encode the same amino acid as encoded by SEQ m NOS: 1 and 3. Such modifications made at the nucleic acid level include, for example, modifications to the nucleotide sequences which are silent or which change the amino acids, e.g. to improve expression or secretion of the protein. Also included are allelic variations, caused by the natural degeneracy of the genetic code.

Also useful in the present invention are mutants of the *omp85* sequences, including 5', 3' or internal deletions, which encode proteins that substantially retain the antigenicity of the full-length Omp85 or other proteins or fragments. Such truncated, or deletion, mutants may be expressed by modified nucleic acid sequences for the purpose of affecting the activity of the full-length or wild-type protein.

As described in more detail below, these nucleic acid sequences are useful in the invention. The nucleic acid sequences are also useful in the production of Omp85 proteins and homologs as well as a variety of fusion or other synthetic proteins

The nucleotide sequences may be readily synthesized or may be isolated by conventional uses or polymerase chain reaction or cloning techniques such as those described herein and in conventional texts such as Sambrook et al, cited above. For example, the nucleic acid sequences of the antigen may be prepared or isolated from genomic libraries using DNA primers and probes and PCR techniques. These sequences, fragments thereof, modifications thereto and the full-length sequences may be constructed recombinantly using conventional genetic engineering or chemical synthesis techniques or PCR, and the like by utilizing the information provided herein. Further, such nucleic acid sequences may be conventionally labeled for diagnostic use. Alternatively for use as therapeutic or vaccine components, the nucleic acid sequences may be admixed with a variety of pharmaceutically acceptable carriers, e.g., saline, liposomes, etc. or incorporated into nucleic acid molecules, e.g., plasmids under the regulatory control of sequences which direct expression of the encoded protein in a selected host cell. The nucleic acid sequences may also be delivered to a host as "naked" DNA or in a gene delivery vehicle, such as a recombinant virus, all as described in detail below.

### B. Protein Sequences

The present invention also uses Omp85 proteins and peptides. These proteins are free from association with other contaminating proteins or materials with which they are found in nature. The *Neisseriae* Omp85 antigen has a relative molecular mass of 85kDa as measured by Western immunoblot (See Example 2 and Fig. 2). In one embodiment, the invention uses a gonococcal Omp85 antigen [SEQ ID NO:2] polypeptide of about 792 amino acids, with a signal peptide, having a predicted molecular weight of 85, 842 daltons.

The meningococcal *omp*85 was found to encode a 797 amino acid polypeptide with a predicted molecular weight of 88.5 kDa (Fig. 5). Between amino acid residues 720 and 745, the menigococcal Omp85 varied substantially from gonococcal Omp85, including the insertion of five additional amino acids. The deduced amino acid sequence [SEQ ID NO: 4] of *N*. *meningitidis* Omp85 was revealed by sequence analysis to be 95% identical to *N. gonorrhoeae* Omp85 and 98% similar to gonococcal Omp85 using the BLAST2 algorithm.

The similarities of these two Omp85 proteins to proteins of other microorganisms provides evidence of an immunological role played by these proteins, as well as other potential roles. The D-15 protective surface antigen (D-15-Ag) of *Haemophilus influenzae* and the Oma87 of *Pasteurella multocida* are the only bacterial proteins, which have been previously described, that are similar to the Omp85 amino acid sequence [SEQ ID NO: 2]. This similarity suggested that these proteins played an important role in host-pathogen interactions and have an important function in pathogenesis. The importance of these Omp85 proteins in pathogenesis and immunobiology was demonstrated by the fact that antibody to similar proteins in *H. influenzae* and *P. multocida* were protective. The similarities suggest that the *Neisseriae* Omp85 proteins are likely important immunological targets of the host immune response.

Western blot analysis demonstrated proteins similar to Omp85 in all of the *Neisseriae* tested with anti-Omp85 and three Neisseriae relationship areas. Area A contains the frank pathogens *N. gonorrhoeae* and *N meningitidis* and opportunistic organisms known to cause severe human diseases such as *N. pharyngis* and *N. cinerea.* Area B contains species, such as *N. mucosae* and *N. lactamica,* typically found in the human nasopharynx which are able to cause opportunistic infections in debilitated hosts. Area C consists of commensal/saprophytic organisms, such as *N. flavescens, N. animalis* and *N. denitricans,* which generally do not cause human infections.

All *Neisseriae* species colonize mucosal surfaces. The presence of Omp85-like proteins in numerous pathogenic and commensal organisms suggests it may be involved in establishing or maintaining colonization. The identification of Omp85 proteins in a number of pathogenic and commensal organisms provides evidence that the Omp85 proteins provide functions involved in establishing or maintaining colonization.

Database searches identified genes in a number of pathogens which encode hypothetical proteins similar to Omp85, including genes in *B. abortus, H. pylori,* and *B. burgdorferi.* The proteins encoded by these genes have not yet been characterized. A search of the Omp85 amino acid sequence against the GenBank data base resulted in the identification of a cyanobacterium protein [Kaneto T. et al, DNA Res., 3: 109-136 (1996)] with 35% similarity to the gonococcal Omp85. The cyanobacterium protein was named IAP75 because of its similarity to the 75 kDa chloroplast import associated protein, IAP75 [Schnell DJ et al, Science, 266: 1007-1011 (1994)]. The chloroplast IAP75 was located in the outer membrane of chloroplasts and was one of four outer membrane components of a complex that transports polypeptides. This suggested that Omp85 might be part of a similar transport complex.

The sequences of other proteins from the chloroplast import associated complex were searched against the Gonococcal Genome Sequencing Project Data Base (Dyer and Rowe, 1997). Sequences similar to the chloroplast IAP34 protein [Kessler F. et al, Science, 266: 1035-1039 (1994)] were identified in the gonococcal genome. IAP34 is believed to be a GTP-binding protein and the sequences of highest similarity to the gonococcal homolog were in regions identified as GTP-binding protein motifs. Surface-crosslinkage studies were performed to determine if Omp85 might participate in a system analogous to the IAP complex (data not shown). Studies using the reversible crosslinker DTBP, which crosslinks proteins that are within 11.9Å of each other [Newhall WJ. et al, Infect. Immun., 28: 785-791 (1980)], showed that Omp85 crosslinked with up to five other outer membrane proteins, one of ~34 kDa (IAP34 homolog) (data not shown). These data, which confirmed Omp85 was exposed on the bacterial surface, support the role for Omp85 of participating in a complex analogous with the chloroplast IAP complex. Further characterization of proteins associated with Omp85 in the outer membrane may provide evidence of additional biological functions of these Omp85 proteins.

One of skill in the art using conventional techniques, such as PCR, may readily use the Omp85 sequences provided herein to identify and isolate other similar proteins. Such methods are routine and not considered to require undue experimentation, given the information provided herein.

Antigens of use in the invention may be characterized by immunological measurements including, without limitation, Western blot, macromolecular mass determinations by biophysical determinations, such as SDS-PAGE/staining, high pressure liquid chromatography (HPLC) and the like, antibody recognition assays, T-cell recognition assays, major histocompatibility complex (MHC) binding assays, and assays to infer immune protection or immune pathology by adoptive transfer of cells, proteins or antibodies.

The Omp85 outer membrane antigens of use in the invention (as well as its naturally occurring variants or analogs in other species) may be isolated in the form of a complete intact protein, or a polypeptide or fragment thereof. In one embodiment, Omp85 is isolated by immunoblot procedures according to its respective molecular mass, as described below in the examples. Such isolation provides the antigen in a form substantially free from other proteinaceous and non-proteinaceous materials of the microorganism. The molecules comprising the polypeptides and antigens of use in the invention may be isolated and further purified using any of a variety of conventional methods including, but not limited to: liquid chromatography such as normal or reversed phase, using HPLC, FPLC and the like; affinity chromatography (such as with inorganic ligands or monoclonal antibodies); size exclusion chromatography; immobilized metal chelate chromatography; gel electrophoresis; and the like. One of skill in the art may select the most appropriate isolation and purification techniques.

Alternatively, the amino acid sequences of the proteins of use in the invention may be produced recombinantly following conventional genetic engineering techniques [see e.g., Sambrook et al, cited above and the detailed description of making the proteins below].

### i. Analogs/Modified Antigens

Also useful in the invention are analogs, or modified versions, of the Omp85 protein, or fragments provided herein. Typically, such analogs differ from the specifically identified proteins by only one to four codon changes. Examples include polypeptides with minor amino acid variations from the illustrated partial amino acid sequence of, for example, the gonococcal Omp85 (SEQ ID NO: 2), in particular, conservative amino acid replacements. Conservative replacements are those that take place within a family of amino acids that are related in their side chains and chemical properties. Homologs having at least 90% identity with either SEQ ID NO: 2 or SEQ ID NO: 4 are useful in this invention. Homologs having at least 95% identity with either SEQ ID NO: 2 or SEQ ID NO: 4 are also useful in this invention.

The algorithms used for these calculations are identified above. Based on the sequence information provided herein, one of skill in the art can readily obtain full-length homologs and analogs from other bacterial species.

An antigen useful in the present invention may also be modified to increase its immunogenicity. For example, the antigen may be coupled to chemical compounds or immunogenic carriers, provided that the coupling does not interfere with the desired biological activity of either the antigen or the carrier. For a review of some general considerations in coupling strategies, see Antibodies. A Laboratory Manual Cold Spring Harbor Laboratory, ed. E. Harlow and D. Lane (1988). Useful immunogenic carriers known in the art, include, without limitation, keyhole limpet hemocyanin (KLH); bovine serum albumin (BSA), ovalbumin, purified protein derivative of tuberculin (PPD); red blood cells; tetanus toxoid; cholera toxoid; agarose beads; activated carbon; or bentonite. Useful chemical compounds for coupling include, without limitation, dinitrophenol groups and arsonilic acid. One of skill in the art may readily select other appropriate immunogenic carriers or coupling agents.

The antigen may also be modified by other techniques, such as denaturation with heat and/or SDS.

### ii. Fragments/Deletion Mutants

Further useful in this invention are additional fragments of the Omp85 polypeptides and peptides identified herein. Such fragments are desirably characterized by having a biological activity similar to that displayed by the complete protein, including, e.g., the ability to induce antibodies which can interfere with the binding of the pathogen to its cellular targets (see Example 8). These fragments are from 8 amino acids in length up to fragments encompassing just short of the entire protein. Such fragments represent consecutive amino acids in the protein sequence. Such a fragment may represent an epitope of the protein.

The Omp85 proteins [SEQ ID NOS:2 and 4] useful in the invention may be modified to create deletion mutants, for example, by truncation at the amino or carboxy termini, or by elimination of one or more amino acids. Deletion mutants are also useful in this invention, as are the DNA sequences encoding them.

In yet another embodiment, the Omp85 peptides or polypeptides useful in this invention may be in the form of a multiple antigenic peptide ("MAP", also referred to as an octameric lysine core peptide) construct. Such a construct may be designed employing the MAP system described by Tam, Proc. Natl. Acad. Sci. USA, 85:5409-5413 (1988). This system makes use of a core matrix of lysine residues onto which multiple copies of the same protein or peptide useful in the invention are synthesized as described [D. Posnett et al., J. Biol. Chem., 263(4):1719-1725 (1988); J. Tam, "Chemically Defined Synthetic Immunogens and Vaccines by the Multiple Antigen Peptide Approach", Vaccine Research and Developments, Vol. 1, ed. W. Koff and H. Six, pp. 51-87 (Marcel Deblau, Inc., New York 1992)]. Each MAP contains multiple copies of only one peptide.

Still other modified fragments of Omp85 may be prepared by any number of now conventional techniques to improve production thereof, to enhance protein stability or other characteristics, e.g. binding activity or bioavailability, or to confer some other desired property upon the protein. Other useful fragments of these polypeptides may be readily prepared by one of skill in the art using known techniques, such as deletion mutagenesis and expression.

### iii. Fusion or Multimeric Proteins and Compositions

The Omp85 protein useful in the present invention, or fragments of it, may also be constructed, using conventional genetic engineering techniques as part of a larger and/or multimeric protein or protein compositions. Antigens useful in this invention may be in combination with outer surface proteins or other proteins or antigens of other pathogens, such as those identified above, or various fragments of the antigens described herein may be in combination with each other. In such combination, the antigen may be in the form of a fusion protein. The antigen useful in the invention may be optionally fused to a selected polypeptide or protein derived from other microorganisms. For example, an antigen or polypeptide useful in this invention may be fused at its N-terminus or C-terminus to a polypeptide from another pathogen or to more than one polypeptide in sequence. Polypeptides which may be useful for this purpose include polypeptides identified by the prior art.

Still another fusion protein useful in this invention is provided by expressing the DNA molecule formed by the *omp85* DNA sequence or a fragment thereof fused to DNA fragments that are homologous (between about 25-95% identity) to Omp85. One example of such a protein comprises the amino acid sequence of SEQ ID NO: 2 to which is fused amino acid fragments that are up to 95% identical to that sequence, e.g., from SEQ ID NO: 4, or from any of the above-described homologous proteins. These fragments may be inserted in any order and may contain repeated sequences. The fused fragments may produce a large DNA molecule which expresses a protein which may stimulate a variety of antibody specificities.

These fusion proteins comprising multiple polypeptides of this disclosure are constructed for use in the vaccine compositions of this invention. These fusion proteins or multimeric proteins may be produced recombinantly, or may be synthesized chemically. They also may include the polypeptides of this disclosure fused or coupled to moieties other than amino acids, including lipids and carbohydrates. Further, antigens useful in this invention may be employed in combination with other vaccinal agents described by the prior art, as well as with other species of vaccinal agents derived from other microorganisms. Such proteins are useful in the prevention of diseases caused by a wide spectrum of *Neisseriae* isolates.

A protein composition which may be a preferred alternative to the fusion proteins described above is a cocktail (i.e., a simple mixture) containing different Omp85 proteins or fragments, optionally mixed with different antigenic proteins or peptides of other pathogens. Such mixtures of these proteins or antigenic fragments thereof are likely to be useful in the generation of desired antibodies to a wide spectrum of *Neisseriae* isolates.

### iv. Salts

An antigen useful in the present invention may also be used in the form of a pharmaceutically acceptable salt. Suitable acids and bases which are capable of forming salts with the polypeptides useful in the present invention are well known to those of skill in the art, and include inorganic and organic acids and bases.

### III. Methods of Making Antigens and Nucleic Acid Sequence useful in the Invention

### A. Expression In Vitro

To produce recombinant Omp85 or peptide fragments useful in the invention, the DNA sequences useful in the invention are inserted into a suitable expression system. Desirably, a recombinant molecule or vector is constructed in which the polynucleotide sequence encoding the selected protein, e.g., Omp85 is operably linked to a heterologous expression control sequence permitting expression of the protein. Numerous types of appropriate expression vectors are known in the art for protein expression, by standard molecular biology techniques. Such vectors are selected from among conventional vector types including insects, e.g., baculovirus expression, or yeast, fungal, bacterial or viral expression systems. Other appropriate expression vectors, of which numerous types are known in the art, can also be used for this purpose. Methods for obtaining such expression vectors are well-known. See, Sambrook et al, Molecular Cloning, A Laboratory Manual, 2d edition, Cold Spring Harbor Laboratory, New York (1989); Miller et al, Genetic Engineering, 8:277-298 (Plenum Press 1986) and references cited therein.

Suitable host cells or cell lines for transfection by this method include bacterial cells. For example, the various strains of *E. coli,* e.g., HB101, MC1061, and strains used in the following examples, are well-known as host cells in the field of biotechnology. Various strains of *B. subtilis, Pseudomonas, Streptomyces,* and other bacilli and the like are also be employed in this method.

Mammalian cells, such as human 293 cells, Chinese hamster ovary cells (CHO), the monkey COS-1 cell line or murine 3T3 cells derived from Swiss, Balb-c or NIH mice are used. Another suitable mammalian cell line is the CV-1 cell line. Still other suitable mammalian host cells, as well as methods for transfection, culture, amplification, screening, production, and purification are known in the art. [See, e.g., Gething and Sambrook, Nature, 293:620-625 (1981), or alternatively, Kaufman et al, Mol. Cell. Biol., 5(7):1750-1759 (1985) or Howley et al, U. S. Patent 4,419,446].

Many strains of yeast cells known to those skilled in the art are also available as host cells for expression of the polypeptides useful in the present invention. Other fungal cells may also be employed as expression systems. Alternatively, insect cells such as *Spodoplera frugipedera* (Sf9) cells may be used.

Thus, a method for producing recombinant Omp85 proteins involves transfecting, e.g., by conventional means such as electroporation, a host cell with at least one expression vector containing a polynucleotide useful in the invention under the control of a transcriptional regulatory sequence. The transfected or transformed host cell is then cultured under conditions that allow expression of the protein. The expressed protein is recovered, isolated, and optionally purified from the cell (or from the culture medium, if expressed extracellularly) by appropriate means known to one of skill in the art.

For example, the proteins are isolated in soluble form following cell lysis, or extracted using known techniques, e.g,, in guanidine chloride. If desired, the proteins or fragments useful in the invention are produced as a fusion protein. Such fusion proteins are those described above. Alternatively, for example, it may be desirable to produce fusion proteins to enhance expression of the protein in a selected host cell, to improve purification, or for use in monitoring the presence of the desired protein, e.g., Omp85, in tissues, cells or cell extracts. Suitable fusion partners for the proteins useful in the invention are well known to those of skill in the art and include, among others, β-galactosidase, glutathione-S-transferase, poly-histidine and maltose binding protein.

### B. Expression In Vivo

Alternatively, where it is desired that the Omp85 (whether full-length or a fragment) be expressed *in vivo,* e.g., to induce antibodies, or alternatively where the *omp85* is to be employed as a DNA vaccine, an appropriate vector for delivery is readily selected by one of skill in the art. Exemplary vectors for *in vivo* gene delivery are readily available from a variety of academic and commercial sources, and include, e.g., adeno-associated virus [International patent application No. PCT/US91/03440], adenovirus vectors [M. Kay et al, Proc. Natl. Acad. Sci. USA, 91:2353 (1994); S. Ishibashi et al, J. Clin. Invest., 92:883 (1993)], or other viral vectors, e.g., various poxviruses, vaccinia, etc. Methods for insertion of a desired gene, e.g., Omp85, and obtaining *in vivo* expression of the encoded protein, are well known to those of skill in the art.

### IV. Antibodies useful in the Invention

The present invention also uses antibodies capable of recognizing and binding the isolated, or modified, or multimeric antigens described above, including antibodies derived from mixtures of such antigens or fragments thereof. Certain of the antibodies useful in the invention may be specific to the *N. gonorrhoeae* or *N. meningitidis* Omp85 proteins, by binding to epitopes on the proteins which differ from the former species to the latter species. For example, an antibody specific for *N. gonorrhoeae* may bind an epitope on SEQ ID NO: 2 which is not present in SEQ ID NO: 4, or vice versa. Thus, an *N. gonorrhoeae* Omp85-specific antibody is defined herein as an antibody that binds an Omp85 antigen of *N. gonorrhoeae* only. An *N. meningitidis* Omp85-specific antibody is defined herein as an antibody that binds an Omp85 antigen of *N. meningitidis* only. Alternatively, certain antibodies to these proteins may bind an epitope present on both the *N. gonorrhoeae* and *N. meningitidis* Omp85 proteins. Still other antibodies useful in the invention may bind an epitope on *N. gonorrhoeae* and *N. meningitidis* Omp85, and the same epitope on the other homologous proteins in homologous or heterologous species of bacteria having homologous proteins (described in Part I, B above).

These antibodies are useful in passive vaccine compositions, which vaccines may also be polyvalent, by containing antibodies to antigens of other microorganisms as well as antibodies to the Omp85 proteins useful in the invention.

The antibodies useful in this invention are generated by conventional means utilizing the isolated, recombinant or modified antigens useful in this invention, or mixtures of such antigens or antigenic fragments. For example, polyclonal antibodies are generated by conventionally stimulating the immune system of a selected animal or human with the isolated antigen or mixture of antigenic proteins or peptides, of this disclosure, allowing the immune system to produce natural antibodies thereto, and collecting these antibodies from the animal or human's blood or other biological fluid.

For example, an antibody useful in the invention can be produced by administering to a vertebrate host the antigen or antigenic composition useful in this invention, e.g., Omp85. Preferably a recombinant version of Omp85 (rOmp85) or an Omp85 MAP is used as an immunogen. A suitable polyclonal antibody against the Omp85 antigen may be generated as antisera, such as the Omp85 antisera employed in the examples herein.

Thus, an antibody useful in the invention is isolated by affinity purifying antiserum generated during an infection of a mammal, e.g., a mouse, with *N. gonorrhoeae* or *N. meningitidis,* using as immunoabsorbant the Omp85 antigen identified herein. Similarly, an antibody useful in the invention is isolated by immunizing mice with a purified, recombinant antigen useful in this invention, or a purified, isolated Omp85 protein of native origin.

Monoclonal antibodies (MAbs) directed against Omp85 are also generated. Hybridoma cell lines expressing desirable MAbs are generated by well-known conventional techniques, e.g. Kohler and Milstein and the many known medications thereof. Similarly desirable high titer antibodies are generated by applying known recombinant techniques to the monoclonal or polyclonal antibodies developed to these antigens [see, e.g., PCT Patent Application No. PCT/GB85/00392; British Patent Application Publication No. GB2188638A; Amit et al., Science, 233:747-753 (1986); Queen et al., Proc. Nat'l, Acad, Sci. USA, 86:10029-10033 (1989); PCT Patent Application No. WO90/07861; and Riechmann et al., Nature, 332:323-327 (1988); Huse et al, Science, 246:1275-1281 (1988)a].

Given the disclosure contained herein, one of skill in the art may generate chimeric, humanized or fully human antibodies directed against Omp85 or antigenic fragments thereof by resort to known techniques by manipulating the complementarity determining regions of animals or human antibodies to the antigen useful in the invention. See, e.g., E. Mark and Padlin, "Humanization of Monoclonal Antibodies", Chapter 4, The Handbook of Experimental Pharmacology, Vol. 113, The Pharmacology of Monoclonal Antibodies, Springer-Verlag (June, 1994).

Alternatively, the antigens are assembled as multi-antigenic complexes [see, e.g., European Patent Application 0339695, published November 2, 1989] or as simple mixtures of antigenic proteins/peptides and employed to elicit high titer antibodies capable of binding the selected antigen(s) as it appears in the biological fluids of an infected animal or human.

Further useful in the present invention are anti-idiotype antibodies (Ab2) and anti-anti-idiotype antibodies (Ab3). Ab2 are specific for the target to which anti-Omp85 antibodies useful in the invention bind and Ab3 are similar to Omp85 antibodies (Ab1) in their binding specificities and biological activities [see, e.g., M. Wettendorff et al., "Modulation of anti-tumor immunity by anti-idiotypic antibodies." in Idiotypic Network and Diseases, ed. by J. Cerny and J. Hiernaux J, Am. Soc. Microbiol., Washington DC: pp. 203-229, (1990)]. These anti-idiotype and anti-anti-idiotype antibodies are produced using techniques well known to those of skill in the art. Such anti-idiotype antibodies (Ab2) can bear the internal image of Omp85 or fragments thereof and are thus useful for the same purposes as Omp85 or the fragments.

In general, polyclonal antisera, monoclonal antibodies and other antibodies which bind to the selected antigen (Ab1) are useful to identify epitopes of Omp85 to separate Omp85 and analogs thereof from contaminants in living tissue (e.g., in chromatographic columns and the like), and in general as research tools and as starting material essential for the development of other types of antibodies described above. Anti-idiotype antibodies (Ab2) are useful for binding the same target and thus may be used in place of the original antigen, e.g., Omp85, to induce an immune response. The Ab3 antibodies are useful for the same reason the Ab1 are useful. Other uses as research tools and as components for separation of Omp85 from other contaminants, for example, are also contemplated for the above-described antibodies.

For use in diagnostic assays, the antibodies are associated with conventional labels which are capable, alone or in concert with other compositions or compounds, of providing a detectable signal. Where more than one antibody is employed in a diagnostic method, the labels are desirably interactive to produce a detectable signal. Most desirably, the label is detectable visually, e.g. colorimetrically. A variety of enzyme systems have been described in the art which will operate to reveal a colorimetric signal in an assay. As one example, glucose oxidase (which uses glucose as a substrate) releases peroxide as a product. Peroxidase, which reacts with peroxide and a hydrogen donor such as tetramethyl benzidine (TMB) produces an oxidized TMB that is seen as a blue color. Other examples include horseradish peroxidase (HRP) or alkaline phosphatase (AP), and hexokinase in conjunction with glucose-6-phosphate dehydrogenase which reacts with ATP, glucose, and NAD+ to yield, among other products, NADH that is detected as increased absorbance at 340 nm wavelength. Other label systems that may be utilized in the methods described herein detectable by other means, e.g., colored latex microparticles [Bangs Laboratories, Indiana] in which a dye is embedded may be used in place of enzymes to form conjugates with the antibodies and provide a visual signal indicative of the presence of the resulting complex in applicable assays. Still other labels include fluorescent compounds, radioactive compounds or elements. Detectable labels for attachment to antibodies useful in diagnostic assays of this disclosure may be easily selected from among numerous compositions known and readily available to one skilled in the art of diagnostic assays. The methods and antibodies are not limited by the particular detectable label or label system employed.

### V. Therapeutic and Vaccine Compositions

### A. Protein-Containing Therapeutic and Vaccine Compositions

The antigens and antibodies useful in the invention, alone or in combination with other antigens and antibodies of, or directed to, other pathogenic microorganisms may be used in vaccine compositions and in methods for treating humans with non-symptomatic infection or symptomatic disease caused by *N. gonorrhoeae, N. meningitidis* or the other pathogens identified above.

For example, one such therapeutic composition may be formulated to contain a carrier or diluent and one or more of the anti-Omp85 antibodies. In compositions containing the Omp85 antigen or antibodies thereto, i.e., protein components, suitable pharmaceutically acceptable carriers may be employed that facilitate administration of the proteins but are physiologically inert and/or nonharmful.

A variety of such pharmaceutically acceptable protein carriers, and/or components suitable for administration therewith may be selected by one of skill in the art. For example, pharmaceutical carriers include, without limitation, sterile saline, lactose, sucrose, calcium phosphate, gelatin, dextran, agar, pectin, peanut oil, olive oil, sesame oil, and water. Additionally, the carrier or diluent may include a time delay material, such as glycerol monostearate or glycerol distearate alone or with a wax. In addition, slow release polymer formulations can be used. Liposomes or liposomal-like vehicles may also be employed.

Optionally, these compositions may also contain conventional pharmaceutical ingredients, such as preservatives, or chemical stabilizers. Suitable ingredients which may be used in a therapeutic composition in conjunction with the antibodies include, for example, casamino acids, sucrose, gelatin, phenol red, N-Z amine, monopotassium diphosphate, lactose, lactalbumin hydrolysate, and dried milk.

Alternatively, or in addition to the antigens or antibodies useful in the invention, other agents useful in treating the disease in question, e.g., antibiotics or immunostimulatory agents and cytokine regulation elements, are expected to be useful in reducing or eliminating disease symptoms. Such agents may operate in concert with the vaccine compositions of this invention. The development of vaccine compositions containing these agents is within the skill of one in the art in view of the teachings of this invention.

Additionally; the vaccine compositions may be polyvalent. Such compositions may contain therapeutic components from other bacterial or viral pathogens, e.g., components of bacterial species homologous to *Neiserriae* or heterologous thereto and/or antigens from a disease-causing virus. Depending upon the compatibility of the components, the Omp85 antibodies or antigens useful in the invention may thus be part of a multi-component vaccine composition directed at more than a single disease.

A therapeutic method involves treating a human for infection with *N. gonorrhoeae* or *N. meningitidis* by administering an effective amount of such a therapeutic composition. An "effective amount" of a proteinaceous composition may be between about 0.05 to about 1000 µg/ml of an antibody or antigen. A suitable dosage may be about 1.0 ml of such an effective amount. Such a composition may be administered 1 - 3 times per day over a 1 day to 12 week period. However, suitable dosage adjustments for protein or nucleic acid containing compositions may be made by the attending physician or veterinarian depending upon the age, sex, weight and general health of the human patient. Preferably, such a composition is administered parenterally, preferably intramuscularly or subcutaneously. However, it may also be formulated to be administered by any other suitable route, including orally or topically. The selection of the route of delivery and dosage of such therapeutic compositions is within the skill of the art.

In another embodiment, the Omp85 antigens, antibodies, and fragments, useful in the invention alone or in combination with other antigens, antibodies, and fragments from other microorganisms, may further be used in compositions directed to induce a protective immune response in a subject to the pathogen. These components of the present invention are also useful for inducing a protective immune response in humans against infection with *N. gonorrhoeae, N. meningitidis* or the other pathogens identified above.

In one embodiment, an outer membrane protein antigen-based vaccine for the prevention of non-symptomatic gonococcal infection or symptomatic disease, non-symptomatic meningococcal infection and symptomatic disease, and other diseases in humans is provided which contains an effective amount of an Omp85 antigen, useful in the invention and a pharmaceutically acceptable carrier or diluent. This vaccine composition may contain one or more of the isolated, recombinant, modified or multimeric forms of the Omp85 antigen useful in the invention, or mixtures thereof Similarly, salts of the antigenic proteins may be employed in such compositions.

In another embodiment of this invention, a polyvalent vaccine composition may include not only the Omp85 antigen or immunogenic fragment thereof, but may also include antigens from other disease-causing agents. Such other agents may be antigens from other *Neisseriae* strains. Such other agents may be antigens from completely distinct bacterial pathogens or from viral pathogens. Combinations of the antigen(s) of this disclosure with other antigens or fragments thereof are also encompassed by this invention for the purpose of inducing a protective immune response in the vaccinated subject to more than a single pathogen. The selection of these other vaccine components is not a limitation of the present invention and may be left to one of skill in the art.

Such proteinaceous vaccines may include exemplary carriers as described above for therapeutic compositions. Optionally, the vaccine compositions may optionally contain adjuvants, preservatives, chemical stabilizers, as well as other conventionally employed vaccine additives. Typically, stabilizers, adjuvants, and preservatives are optimized to determine the best formulation for efficacy in the target human. Suitable exemplary preservatives include chlorobutanol, potassium sorbate, sorbic acid, sulfur dioxide, propyl gallade, the parabens, ethyl vanillin, glycerin, phenol, and parachlorophenol.

With regard to the adjuvant, one or more of the above described vaccine components may be admixed or adsorbed with a conventional adjuvant. The adjuvant is used to attract leukocytes or enhance an immune response. Such adjuvants include, among others, RIBI adjuvant, mineral oil and water, aluminum hydroxide, AMPHIGEN adjuvant, ADJUVAX adjuvant, AVRIDINE adjuvant, L121/squalene, D-lactide-polylactide/glycoside, pluronic plyois, muramyl dipeptide, killed *Bordetella,* and saponins, such as Quil A.

The invention thus can be used in a prophylactic method entailing administering to a human an effective amount of such a composition. The protein antigenic compositions are administered in an "effective amount", that is, an amount of antigen that is effective in a route of administration to provide a vaccinal benefit, i.e., protective immunity. Suitable amounts of the antigen can be determined by one of skill in the art based upon the level of immune response desired. In general, however, the vaccine composition contains between 1 ng to 1000 mg antigen, and more preferably, 0.05 µg to 1 mg per ml of antigen. Suitable doses of the vaccine composition of the invention can be readily determined by one of skill in the art. Generally, a suitable dose is between 0.1 to 5 ml of the vaccine composition. Further, depending upon the human patient being treated, i.e. its weight, age, and general health, the dosage can also be determined readily by one of skill in the art.

In general, the vaccine can be administered once; and optionally boosters can be administered periodically thereafter. The vaccine may be administered by any suitable route. However, parenteral administration, particularly intramuscular, and subcutaneous, is the preferred route. Also preferred is the oral route of administration. Routes of administration may be combined, if desired, or adjusted.

In still another vaccine embodiment, the invention includes a composition which delivers passive protection against infection by the pathogen. For this composition, the antibodies against the Omp85 proteins disclosed herein are useful to provide to the subject a short-term, passive immune protection against infection. These passive immunity vaccine compositions may contain antibodies to other pathogens and suitable vaccine additives as described above, e.g., adjuvants, etc. These compositions may be administered in dosages similar to those described above for the compositions which actively induce immune protection in the vaccinated subject.

### B. Nucleic Acid Containing Compositions

The nucleic acid sequences or anti-sense sequences useful in the invention, alone or in combination with other nucleic acid sequences encoding antigens or antibodies of, or directed to other pathogenic microorganisms may further be used in therapeutic compositions and in methods for treating humans with the disease caused by infection with *N. gonorrhoeae, N. meningitidis* or the other pathogens identified above. In another embodiment, the nucleic acid sequences useful in the invention, alone or in combination with nucleic acid sequences encoding other antigens or antibodies from other pathogenic microorganisms, may further be used in compositions directed to actively induce a protective immune response in a subject to the pathogen. These components of the present invention are useful in inducing a protective immune response in humans against infection with *N. gonorrhoeae, N. meningitidis* or the other pathogens identified above.

For use in the preparation of the vaccine compositions, nucleic acid delivery compositions and methods are useful, which are known to those of skill in the art, The *omp85* sequences or fragments thereof may be employed in the uses of this invention or in the compositions described herein as DNA sequences, either administered as naked DNA, or associated with a pharmaceutically acceptable carrier and provide for *in vivo* expression of the Omp85 protein or peptide. So-called 'naked DNA' may be used to express the Omp85 protein or peptide fragment (or anti-sense sequences) *in vivo* in a patient. [See, e.g., J. Cohen, Science, 259:1691-1692 (March 19, 1993); E. Fynan et al, Proc Natl Acad. Sci., 90: 11478-11482 (Dec. 1993); J. A. Wolff et al, Biotechniques, 11:474-485 (1991) which describe similar uses of 'naked DNA'. For example, "naked" *omp85* DNA (or anti-sense sequences) associated with regulatory sequences may be administered therapeutically or as part of the vaccine composition e.g., by injection.

Alternatively, *omp85* DNA or anti-sense DNA may be administered as part of a vector or as a cassette containing the Omp85-encoding DNA sequences or fragments or anti-sense sequences thereof operatively linked to a promoter sequence and other plasmid sequences. Briefly, the DNA encoding the Omp85 protein (or anti-sense sequence) or desired fragment thereof may be inserted into a nucleic acid cassette. This cassette may be engineered to contain, in addition to the *omp85* sequence to be expressed (or anti-sense sequence), other optional flanking sequences which enable its insertion into a vector. This cassette may then be inserted into an appropriate DNA vector downstream of a promoter, an mRNA leader sequence, an initiation site and other regulatory sequences capable of directing the replication and expression of that sequence *in vivo.* This vector permits infection of vaccinate's cells and expression of the *omp85* (or anti-sense sequence) *in vivo.*

Numerous types of appropriate vectors are known in the art for protein expression and may be designed by standard molecular biology techniques. Such vectors are selected from among conventional vector types including insects, e.g., baculovirus expression, or yeast, fungal, bacterial or viral expression systems. Methods for obtaining such vectors are well-known. See, Sambrook et al, Molecular Cloning. A Laboratory Manual, 2d edition, Cold Spring Harbor Laboratory, New York (1989); Miller et al, Genetic Engineering, 8:277-298 (Plenum Press 1986) and references cited therein. Recombinant viral vectors, such as retroviruses or adenoviruses, are preferred for integrating the exogenous DNA into the chromosome of the cell.

Also where desired, the regulatory sequences in such a vector which control and direct expression of the *omp85* gene product or anti-sense sequence in the transfected cell include an inducible promoter. Inducible promoters are those which "turn on" expression of the gene when in the presence of an inducing agent. Examples of suitable inducible promoters include, without limitation, the sheep metallothionine (MT) promoter, the mouse mammary tumor virus (MMTV), the tet promoter, etc. The inducing agents may be a glucocorticoid such as dexamethasone, for, e.g., the MMTV promoter, or a metal, e.g., zinc, for the MT promoter; or an antibiotic, such as tetracycline for tet promoter. Still other inducible promoters may be selected by one of skill in the art, such as those identified in International patent application WO95/13392, published May 18, 1995. The identity of the inducible promoter is not a limitation of this invention.

When *omp85* nucleic acid sequences or anti-sense sequences are employed as the therapeutic agent or vaccine agent as 'naked DNA' operatively linked to a selected promoter sequence, rather than the protein itself, the amounts of DNA to be delivered and the routes of delivery may parallel the protein amounts for vaccine or therapeutic delivery described above and may also be determined readily by one of skill in the art.

Thus, as one preferred example, a therapeutic composition may be formulated to contain a carrier or diluent and one or more plasmid or DNA molecule or recombinant virus containing a nucleic acid sequence which is anti-sense to the *omp85* gene sequence [SEQ ID NO: 1 or 3], a fragment thereof, under control of suitable sequences regulating the expression thereof. In compositions containing the anti-sense *omp85* nucleic acid sequences, vehicles suitable for delivery of DNA may be employed.

Additionally, these therapeutic compositions may be polyvalent. Such compositions may contain therapeutic components which are anti-sense sequences of other bacterial or viral origin, e.g., the anti-sense sequence of components of bacterial species homologous to *Neiserriae* or heterologous thereto and/or anti-sense sequence derived from antigens from a disease-causing virus. Depending upon the compatibility of the components, the anti-sense sequences to the Omp85 antigens useful in the invention may thus be part of a multi-component therapeutic composition directed at more than a single disease.

A therapeutic method involves treating a human for infection with *N. gonorrhoeae* or *N. meningitidis* by administering an effective amount of such a therapeutic nucleic-acid containing composition. An "effective amount" of a nucleic acid composition may be calculated as that amount capable of expressing *in vivo* the above effective amounts of exogenously delivered proteins. Such amounts may be determined by one of skill in the art. Preferably, such a composition is administered parenterally, preferably intramuscularly or subcutaneously. However, it may also be formulated to be administered by any other suitable route, including orally or topically. The selection of the route of delivery and dosage of such therapeutic compositions is within the skill of the art.

As another example, a vaccine composition of this invention may be a DNA vaccine, which includes the *omp85* DNA sequence [SEQ ID NOS: 1 or 3] or a fragment thereof which encodes an immunogenic protein or peptide, optionally under the control of regulatory sequences. In one embodiment, this vaccine composition may contain a nucleic acid sequence that encodes one or more of the isolated, recombinant, modified or multimeric forms of the Omp85 antigen useful in the invention, or mixtures thereof.

In another embodiment of this invention, polyvalent vaccine compositions may include not only the nucleic acid sequence encoding the Omp85 antigen or an immunogenic fragment thereof, but may also include nucleic acid sequences encoding antigens from other disease-causing agents. Such other agents may be antigens from other *Neisseriae* strains. Such other agents may be nucleic acid sequences encoding antigens from completely distinct bacterial pathogens or from viral pathogens. Combinations of the antigen-encoding sequences useful in this invention with other nucleic acid sequences encoding antigens or fragments thereof from other pathogens are also encompassed by this invention for the purpose of inducing a protective immune response in the vaccinated subject to more than a single pathogen. The selection of these other vaccine components is not a limitation of the present invention, and may be left to one of skill in the art.

Such "DNA" or nucleic acid vaccines may include exemplary carriers as described above for therapeutic compositions and, where suitable, the components or additives described above with reference to the proteinaceous compositions, e.g. adjuvants, preservatives, chemical stabilizers, etc. as well as other conventionally employed vaccine additives. Additives suitable for use in nucleic acid compositions are known to those of skill in the art, including certain lipids and liposomes, among other known components.

Generally, a suitable nucleic acid-based treatment contains between 1x10⁻³ plaque forming unit (pfu) to 1x10¹² pfu per dose, if a virus is the delivery vector. Otherwise, the dosage is adjusted to provide the same amount of expressed protein as is provided by the protein vaccines. However, the dose, timing and mode of administration of these compositions may be determined by one of skill in the art. Such factors as the age, and physical condition of the vaccinate may be taken into account in determining the dose, timing and mode of administration of the immunogenic or vaccine composition of the-invention.

The following examples are provided to illustrate the invention and do not limit the scope thereof.

### EXAMPLE 1: Bacterial Strains, Cells, Methods and Culture Conditions

*N. gonorrhoeae* strains FA19, FA635, FA1090, JS1, F62 and MS11LosA used in the examples below were provided by Dr. William M. Shafer (Emory University, Atlanta, GA) Dr. John Swanson (Rocky Mountain Laboratories, Hamilton, MT) or the American Type Culture Collection (Rockville, MD). *N. meningitidis* strains MP78, MP3, MP81, and HH were provided by Dr. Mark S. Peppler (University of Alberta, Edmonton, Alberta, Canada) and Dr. Zell McGee (University of Utah, Salt Lake City, UT). All other strains were acquired from the American Type Culture Collection.

*Moraxella catarrhalis* (ATCC# 8193) and Neisserial strains were grown on clear gonococcal typing media [Swanson J., Infect. Immun., 19: 320-331 (1978)]. *E*. *coli* and all other Gram negative strains were grown an Luria broth.

*E. coli* XL1-Hlue and SOLR cells were obtained from Stratagene Cloning Systems (La Jolla, CA). DNA fragments were purified from agarose gels using Gene Clean II (Bio101, La Jolla, CA). Plasmids were purified using the QIAprep quick spin kit (Qiagen, Chatsworth, CA). Immunoblotting was done with Millipore (Bedford, MA) Immobilon PVDF. Unless specified otherwise in the examples below, all reagents were obtained from Sigma Chemical Co. (St. Louis, MO).

### EXAMPLE 2: Cloning and Immunological Screening of a Gonococcal Genomic Library

A genomic DNA library was produced by purifying *N. gonorrhoeae* strain FA19 genomic DNA and partially digesting the DNA into fragments with the restriction endonuclease *Tsp*509 [New England Biolabs]. These DNA fragments were ligated with T4 DNA ligase [New England Biolabs] into the *Eco*RI restriction site of the lambda Zap II bacteriophage vector [Stratagene Cloning Systems, La Jolla, CA]. The ligated phage DNA was packaged, and plated on *E. coli* DH5α [Gibco BRL, Gaithersburg, MD] host cells. The resulting library was screened immunologically for the expression of gonococcal surface proteins according to protocols provided with the Lambda ZapII vector system. The plaques from the library were screened with anti-GC-OM, an antiserum raised to isolated gonococcal outer membranes.

Plasmid DNA was rescued from phage producing immunoreactive plaques. The plasmid, pDR4, contained a 2.6 kbp gonococcal DNA insert. The gonococcal DNA in pDR4 was subcloned into pUP 1 [Elkins C., J. Bacteriol., 173: 3911-3913 (1991), generously provided by Dr. Chris Elkins (University of North Carolina, Chapel Hill, NC)] yielding pOmp85.

The cloned gonococcal DNA fragment in pOmp85 was characterized by restriction enzyme analysis. The fragment contained three internal *Hinc*II restriction sites which allowed the subcloning of three fragments into pBluescript [Stratagene Cloning Systems La Jolla, CA]. These fragments and pDR4 were sequenced by the University of Montana Molecular Biology Facility. A gene-walking strategy was used to sequence those regions which could not be sequenced with universal vector primers. The entire length of the gonococcal DNA fragment was sequenced at least twice and most of the DNA was sequenced from both strands. The deduced amino acid sequence and comparisons of Omp85 homologs were obtained with the MacVector software package (Eastman Chemical Co., New Haven, CT).

The 2.6 kb gonococcal DNA in pOmp85 was found to contain a large open reading frame (ORF) which encoded a polypeptide of 792 amino acids with a predicted molecular weight of 87.8 kDa (Fig. 2) [SEQ ID NO:2]. The polypeptide contained a putative signal peptide [Von Heijne G., Nuc. Acids Res., 14: 4683-4690 (1986)]. Removal of this signal peptide yielded a mature polypeptide with an 85.8 kDa predicted molecular weight. The polypeptide also possesses a carboxyl-terminal phenylalanine residue characteristic of outer membrane proteins [Struyve M. et al, J. Mol. Biol., 218: 141-148 (1991)]. A ribosome-binding sequence preceded the initiation codon of the ORF. Potential promoter sequences were not readily apparent. The ORF was preceded and followed by putative rho-independent transcriptional stop sites. The approximately 85 kDa gonococcal protein expressed by pOmp85 was designated Omp85 and the gene encoding it was designated *omp*85.

### EXAMPLE 3: Cloning and Sequencing of Meningococcal omp85

The meningococcal *omp*85 was obtained by PCR amplification using the Boehringer Mannheim (Indianapolis, IN) Expand High Fidelity PCR System. The design of PCR primers was based on gonococcal *omp*85 and flanking sequences. The positive-sense *omp*85 PCR primer contained the first five codons of the gonococcal *omp*85 with an EcoRI restriction site and two extra nucleotides added to the 5' end (CGGAATTCATGAAACTGAAACAG) [SEQ ID NO: 5]. The negative-sense *omp*85 PCR primer contained the reverse-compliment of six codons (TTGCAGTTTTTGCAATTC) [SEQ ID NO: 6] of the gonococcal *omp*H sequence located 244 base pairs 3' of *omp*85 termination codon. These primers were used in a PCR reaction with purified *N. meningitidis* HH DNA as template. The meningococcal *omp*85 PCR product was ligated into pUP1 to yield pMCOmp85. The sequence of the meningococcal *omp*85 was obtained essentially as described for the gonococcal *omp*85.

The meningococcal *omp*85 was found to encode a 797 amino acid polypeptide with a predicted molecular weight of 88.5 kDa (Fig. 5). The meningococcal Omp85 was 95% identical and 98% similar to gonococcal Omp85. Between amino acid residues 720 and 745, the menigococcal Omp85 varied substantially from gonococcal Omp85, including the insertion of five additional amino acids.

### EXAMPLE 4: Presence of Omp85 in Strains of N. gonorrhoege and N. meningitidis

### A. Western Analysis

The Western blot analyses were performed as follows. Bacterial proteins were separated by SDS-PAGE (12.5%) [Laemmli UK, Nature, 227: 680-695 (1970)]. The separated proteins were electrophoretically transferred onto PVDF as previously described [Judd RC., Anal. Biochem., 173: 307-316 (1988)]. Blotting was performed in 20mM sodium phosphate buffer, pH 8.0 for 2 hr at 600 mA. The PVDF membrane was blocked for 1 hr with PBS Tween at room temperature, incubated overnight in anti-GC-OM or anti-Omp85 sera at 4°C, washed several times, incubated with protein A-horseradish peroxidase conjugate (Boehringer Mannheim, Indianapolis, IN) and developed with 4-chloro-1-naphthol.

Western blot analysis of the proteins from *E. coli* DH5α/pOmp85 were performed by separating bacterial cell lysates by SDS-PAGE, staining with Coomassie Brilliant blue (CBB) or transferring the lysates to membranes and probing with anti-GC-OM serum. The results revealed expression of an approximately 85 kDa polypeptide that was reactive with the anti-GC-OM serum (Fig. 1).

Anti-GC-OM serum was reacted in Western blot analysis with total cell proteins from six representative strains of *N*. *gonorrhoene* and four strains of *N. meningitidis.* Whole cell lysates of *E*. *coli* DH5α, *E. coli* DH5α/pOmp85, and *N. gonorrhoeae* strains FA19, FA635, FA1090, JS1, F62 and MS11LosA and *N. meningitidis* strains MP78, MP3. MP81 and HH were separated by 12.5% SDS-PAGE, blotted and probed with the anti-GC-OM serum. An immunoreactive, approximately 85 kDa protein band was detected in all strains of *N. gonorrhoeae* and *N. meningitidis* (Fig. 3), but not in the control *E. coli* DH5α.

### B. Southern Analysis

The Southern analyses are performed as follows. Chromosomal DNA was obtained by phenol extraction [Moore D, "Preparation and analysis of DNA", in: Ausubel FM, et al, eds. Current Protocols in Molecular Biology. New York: John Wiley and Sons, (1997): 2.1.1-2.1.3] and then digested with various endonucleases. Digested DNA was electrophoretically separated on a 1% agarose gel and the DNA transferred to nitrocellulose with the Bio-Rad Model 785 Vacuum Blotter according to instructions and Southern [Southern E., J. Mol. Biol., 98: 503-510 (1975)]. Probe DNA was extracted from agarose gels using Bio 101 glassmilk (Vista, CA). The probe was labeled and the blot probed with the Amersham ECL system (Arlington Heights, IL).

A Southern blot (Fig. 4) illustrated the identification of *omp*85 in *N. gonorrhoeae* and *N. meningitidis* by Southern analysis. Genomic DNA from *E. coli* DH5α, *N. gonorrhoeae* FA19 and *N. meningitidis* strains MP3, MP73, MP81 and HH were digested with restriction endonucleases (*Hinc*II, *Eco*RI, *Pst*I, *Cla*I). Blots of the separated DNA digests were probed with a 688 bp fragment of gonococcal *omp*85 that extended from the most 3' *Hinc*II site of *omp*85 to a *Hinc*II site in the vector near the 3' end of the cloned gene. This fragment was used as a positive control.

In *N. gonorrhoeae* strain FA19 and in all of the *N. meningitidis* strains, the *omp85* probe hybridized with a single DNA band. A single band was also identified in gonococcal strains FA635, FA1090, JS1, F62, and MS11 (data not shown). These results suggested that *omp85* was conserved as a single copy in pathogenic Neisseria. Sequence data indicated that *Hinc*II cleaved *omp*85 internally at three sites. The probe hybridized to a fragment larger than itself in the *Hinc*II digest of gonococcal DNA (FA19 *- Hinc*II). The genomic fragment resulted from an internal and external *Hinc*II cleavage; the probe lacked the flanking gonococcal sequence containing the external *Hinc*II site. The genomic *Hinc*II band that hybridized to the probe was probably derived from a single copy of the *omp*85 gene since it is unlikely that duplicate copies of the gene would have identically-located *Hinc*II sites, generating fragments of identical size.

The enzyme *Pst*I cleaved 309 base pairs from the 5' end of *omp*85. The single fragment of *Pst*I-digested gonococcal genomic DNA (FA19 - *Pst*I) which hybridized with the probe probably contained a single copy of the gene. It is possible, but unlikely, that the *Pst*I fragments contained approximately 2 kbp segments of *omp*85 in inverted orientations at each end of the approximately 8 kbp fragment. Sequence data indicated that the enzymes *Cla*I and *Eco*RI did not cleave within *omp*85. The *omp*85 probe hybridized to single bands of similar size in the *Cla*I digested DNA of both *N. gonorrhoeae* and *N. meningitidis.* These bands suggested a single copy of *omp85,* since it is unlikely that bands this small (<6 kbp) contained two copies of the approximately 2.3 kbp gene and it is unlikely that the bands represent fragments of identical size from duplicate copies of the gene. Similar results were obtained for gonococcal strains FA635, FA1090, JS1, F62 and MS11 (data not shown). These results support the conclusion that *omp85* is conserved as a single copy in pathogenic Neisseria.

### EXAMPLE 5: Similarity to Known Proteins

The non-redundant Genbank CDS database was searched [Altchul SD. et al, J. Mol Biol., 215: 403-407 (1990)] for proteins similar to gonococcal Omp85. The *H. influenzae* D-15-Ag was 31.5% identical and 61.4% similar (identical plus conserved) to gonococcal Omp85. The *P. multocida* Oma87 was 31.6% identical and 61.3% similar to Omp85. Several hypothetical proteins with similarity to Omp85 were identified. A *Brucella abortus* hypothetical protein (Genbank accession #U51683, Bearden, *et al.,* unpublished) was 24.3% identical and 54.2% similar. A hypothetical *E. coli* protein (Genbank accession #U70214, Schramm, *et al.,* unpublished) was 33% identical and 62% similar to Omp85. The gonococcal Omp85 was also similar to hypothetical proteins of *Helicobacter pylori* (Genbank assession # AE001178 - [Tomb JF. et al., Nature, 388: 539-547 (1997)]), 23% identical and 51% similar, and *Borrelia burgdorferi* (Genbank assession #AE001178 - [Fraser CM. et al, Nature, 390: 580-586 (1997)]), 18% identical and 46% similar.

### EXAMPLE 6: Gene Organization and Flanking Genes

Several hundred base pairs of the DNA flanking the *omp85* ORFs of both *N. gonorrhoeae* and *N. meningitidis* were sequenced. For both *N. gonorrhoeae* and *N. meningitidis,* a gene similar to *omp*H of *Salmonella typhimurium* [Kosk P. et al, J. Biol Chem., 264:18973-18980 (1989)]was identified approximately 65 base pairs 3' of the *omp85* ORF. A gene similar to *omp*H has also been identified in the same relative position in *H. influenzae* [Fleischmann, RD et al, Science, 269: 496-512 (1995)] and *P. multocida.* In *H. influenzae,* a gene encoding a hypothetical protein, HI0918, was located 5' of the D-15-Ag gene [Fleischmann, RD et al, Science, 269: 496-512 (1995)]. A gene homologous to the HI0918 gene was identified at the same relative position in *N. gonorrhoeae.* These results indicated that the gene arrangement around *omp*85 homologs was notably conserved.

### EXAMPLE 7: Presence in Neisseriae and Other Species

Western blot analysis was used to determine if proteins similar to Omp85 were produced by commensal Neisseriae and by other Gram negative species. To allow more specific immunological analysis, an Omp85-specific polyvalent rabbit sera was produced through use of a fusion protein in which the first 200 amino acids of the gonococcal Omp85 were genetically fused to maltose binding protein (MBP).

### A. Production of a MBP/Omp85 fusion protein

Fragments of gonococcal Omp85 were genetically fused to MBP, affinity purified and used to produce Omp85-specific antiserum. *Tsp*509I digested *omp*85 from pOmp85 was ligated into the *Eco*RI digested, maltose binding protein fusion vector, pMAL-c2 [New England Biolabs, Beverly, MA]. Sequence analysis had revealed that this could result in the fusion of several different omp85 fragments in frame with *mal*E in pMAL-c2. The ligated DNA was transformed into *E. coli* DH5α and the transformants were screened for the expression of Omp85 antigens with the anti-OM serum. A number of immunoreactive clones were identified and characterized. The plasmid in the most immunoreactive of these was designated pMO4 and determined by sequence analysis to express a fusion ofMBP with the first 181 amino acids of Omp85. The MBP/Omp85 fusion protein was affinity purified from *E*. *coli* DH5α/pMO4 as previously described [Marchion DC et al, Mol Microbiol., 6: 231-240 (1997)].

### B. Raising of Anti-sera

Purified MBP/Omp85 was used to raise an anti-Omp85 sera in New Zealand white rabbits. The rabbits were initially immunized with 1 mg of protein in Freund's complete adjuvant administered subcutaneously. Two weeks later 1 mg was administered subcutaneously in Freund's incomplete adjuvant. The rabbits then received intravenous injections of 0.1 mg every two weeks for eight weeks. Sera was collected and absorbed (1:1) with a lysed culture of *E. coli*/pMal-c2 induced with 1 mM IPTG for 1 hour. The resulting rabbit serum was designated anti-Omp85.

### C. Western Analysis of representative strains of N. gonorrhoeae and N. meningitidis

The anti-Omp85 serum was used to probe Western blots containing cell lysates of *E. coli* DH5α, *E. coli* DH5α/pOmp85 and representative strains of *N. gonorrhoeae* and *N. meningitidis* (Fig. 6). Whole cell lysates of *E. coli* DH5α, *E. coli* DH5α/pOmp85, *N. gonorrhoeae* strains FA19, FA635, FA1090, JS1, F62 and MS11LosA, *N. meningitidis* strains MP78, MP3, MP81 and HH, and *E coli* DH5α/pMCOmp85 were separated by 12.5% SDS-PAGE, blotted and probed with the anti-Omp85 serum.

The anti-Omp85 serum reacted with the recombinant Omp85 produced by *E. coli* DH5α/pOmp85 and with proteins of approximately 85 kDa in all of the *N. gonorrhoeae* and *N. meningitidis* strains tested. These results indicated that Omp85 was conserved in the pathogenic Neisseriae. Presorption of the antiserum removed the majority of non-specific antibodies, but complete removal of all reactive antibody is not possible, thus, some reactive bands can be seen in the *E. coli* lanes of Fig. 6. Reactive bands in the 35kDa-42kDa range in *N. gonorrhoeae* lanes are Por proteins, which non-specifically bind protein-A. Meningococcal Pors do not bind protein-A as strongly (data riot shown).

### D. Western Analysis of Commensal Nesseriae and Other Gram Negative Species

The anti-Omp85 was also used to probe Western blots containing cell lysates of commensal Nesseriae and several other Gram negative species. Whole cell lysates of *E*. *coli* DH5α, *E. coli* DH5α/pOmp85, *N, gonorrhoeae* FA19 (A), *Neisseria pharyngis* (A), *Neisseria cinerea* (A), *Neisseria lactamica* (B), *Neisseria mucosae* (B), *Neisseria flavescens* (C), *Neisseria animalis* (C), *Neisseria denitrificans* (C), *Moraxella cotarrhalis* (D), *Klebsiella pneumoniae, Pseudomonas aeniginosa,* and *N. meningitidis* HH (A) were separated in a 12.5% SDSαPAGE gel, blotted and probed with anti-Omp85. In a separate experiment, whole cell lysates of *E*. *coli* DH5α, *E. coli* DH5α/pOmp85, *N. gonorrhoeae* FA19, *Salmonella typhimitritim, Shigella flexn*e*ri*, *E. coli* strains 35150 (enterohemorrhagic - EHEC), 35401 (enterotoxigenic - ETEC), 43887 (enteropathogenic - EPEC), 43892 (enteroinvasive - EIEC) and *N. meningitidis* were separated in a 12.5% SDS-PAGE gel, blotted and probed with anti-Omp85.

Two SDS-PAGE (Figs. 7A and 7B) illustrate the distribution of Omp85 in pathogenic and commensal Neisseria (relationship areas A, B, C, and D) and related Gram negative bacteria. Omp85 homologs were identified in all of the commensal Neisserial species tested. This suggested that Omp85 was conserved among all the Neisserial species. The anti-Omp85 serum failed to identify any Omp85 homologs in *Moraxella catarrhalis* which is closely related to the Neisseriae [Rossau R. et al, Int. J. Syst. Bacteriol., 39: 185-198 (1989)]. Southern analysis confirmed the absence of an *omp*85 homolog in this species (data not shown). The serum failed to identify any Omp85 homologs in *Klebsiella pneumoniae, Pseudomonas aeruginosa, Salmonella typhimurium, Shigella flexneri* and four pathogenic strains of *E*. *coli* tested.

### EXAMPLE 8: Gonococcal Cell Adherence Assay

Gonococcal cell adherence assays were performed to evaluate the effect of antiserum specific for outer membrane protein 85 (Omp85) on the ability of gonococcal strains MS11LOSA (MS11) and FA19 to bind to Chang epithelial cells. Fab fragments were prepared from antiserum to the first 178 amino acids of Omp85 [SEQ ID NO: 2], hyperimmune antiserum to bovine serum albumin (BSA) and normal rabbit serum (NRS) and added at 1 µg, 10 µgs or 100 µgs per ml to wells containing a confluent layer of Chang conjunctiva cells. Approximately 2.5 x 10⁵ bacteria (*N. gonorrhoeae* strain MS 11 or FA19) were added to each well and allowed to adhere for 3 hours. Following fixation and immunogoid/silver staining, the number of adherent gonococci was determined for 22 cells. The lowest and highest numbers were discarded and the average number of bacteria/cell were determined. The resulting data is reported in the bar graph of Fig. 8, indicating that Omp85-specific antibody was able to bind to the surface of the bacteria and interfere with the ability of the bacteria to adhere to the Chang epithelial cells.

## Claims

1. A vaccine composition for the protection of a subject against Neisserial disease comprising, in a pharmaceutically acceptable carrier, an effective amount of a polypeptide selected from the group consisting of
(a) the protein of SEQ ID NO: 2;
(b) the protein of SEQ ID NO: 4;
(c) a peptide fragment of SEQ ID NO: 2 or 4 consisting of eight or more consecutive amino acids thereof;
(d) a protein or peptide sharing at least 90% identity with any of the sequences of (a)-(b); and
(e) a sequence of any of (a) - (d) fused to a second polypeptide or protein.

2. A composition according to claim 1 which comprises more than one protein or peptide of any of (a) - (e).

3. A composition according to claim 1 or 2, further comprising at least one other antigen or fragment thereof from a heterologous or homologous pathogenic bacterial species.

4. A composition according to claim 3 wherein said other antigen is fused to one of (a) - (c).

5. A composition according to any one of the preceding claims, wherein said polypeptide lacks the signal sequence spanning amino acids 1-21 of SEQ ID NO: 2 or 4.

6. A method of manufacturing a composition according to any one of the preceding claims, comprising the steps of isolating a recombinant polypeptide comprising at least eight consecutive amino acids from the amino acid sequence of SEQ ID NO: 2 or 4, which polypeptide induces antibodies that recognize SEQ ID NO: 2 or 4, and formulating said polypeptide in an amount effective to induce said antibodies in a mammalian subject, with a pharmaceutically acceptable carrier.

7. A vaccine composition for the protection of a subject against Neisserial disease comprising, in a suitable nucleic acid delivery vehicle, an effective amount of a nucleic acid sequence selected from the group consisting of:
(a) SEQ ID NO: 1;
(b) SEQ ID NO: 3;
(c) a fragment of SEQ ID NO: 1 or 3 consisting of 15 or more consecutive nucleic acids thereof;
(d) a sequence encoding a polypeptide sharing at least 90% identity with either SEQ ID NO: 2 or SEQ ID NO: 4, and
(e) a sequence of any of (a)-(d) further encoding a second polypeptide or protein.

8. A composition according to claim 7 which comprises more than one nucleic acid sequence of any of (a) - (e).

9. A composition according to claim 7 or 8, further comprising at least one other nucleic acid sequence encoding an antigen or fragment thereof from a heterologous or homologous pathogenic bacterial species.

10. A composition according to claim 9, wherein said other antigen is fused to one of (a) - (c).

11. A vaccine composition for the protection of a subject against Neisserial disease comprising an isolated antibody that binds to a protein or peptide selected from the group consisting of:
(a) the protein of SEQ ID NO: 2;
(b) the protein of SEQ ID NO: 4;
(c) a peptide fragment of SEQ ID NO: 2 or 4 consisting of eight or more consecutive amino acids thereof; and
(d) a protein or peptide sharing at least 90% identity with any of the sequences of (a)-(b)

12. A composition according to claim 11, comprising a single isolated antibody.

13. A composition according to claim 11, comprising multiple antibodies.

14. Use of a composition according to any of claims 1 to 13 in the manufacture of a medicament for the protection of a subject against Neisserial disease.

## Patentansprüche

1. Vakzinzusammensetzung zum Schutz eines Subjekts gegen Neisseria-Erkrankung umfassend in einem pharmazeutisch annehmbaren Träger eine wirksame Menge eines Polypeptids ausgewählt aus der Gruppe bestehend aus:
(a) dem Protein gemäß Seq. ID Nr. 2;
(b) dem Protein gemäß Seq. ID Nr. 4;
(c) ein Peptidfragment von Seq. ID Nr. 2 oder 4 bestehend aus 8 oder mehr aufeinander folgenden Aminosäuren davon;
(d) ein Protein oder Peptid, das mindestens 90 % Identität mit eine der Sequenzen von (a) bis (b) aufzeigt und
(e) eine Sequenz nach (a) bis (d) fusioniert mit einem zweiten Polypeptid oder Protein.

2. Zusammensetzung nach Anspruch 1, wobei diese ein oder mehrere Proteine oder Peptide nach (a) bis (e) enthält.

3. Zusammensetzung nach Anspruch 1 oder 2, weiterhin enthältend mindestens ein anderes Antigen oder Fragment davon aus einer heterologen oder homologen pathogenen Bakterienart.

4. Zusammensetzung nach Anspruch 3, wobei dieses andere Antigen mit einem von (a) bis (c) fusioniert ist.

5. Zusammensetzung nach zumindest einem der vorherigen Ansprüche, wobei dem Polypeptid die Signalsequenz umfassend Aminosäuren 1 bis 21 der Seq. ID Nr. 2 oder 4 fehlt.

6. Verfahren zur Herstellung einer Zusammensetzung nach zumindest einem der vorherigen Ansprüche, umfassend die Schritte des Isolierens eines rekombinanten Polypeptids umfassend mindestens acht aufeinanderfolgende Aminosäuren der Aminosäuresequenz von Seq. ID Nr. 2 oder 4, das Polypeptid induziert Antikörper, die die Seq. ID Nr. 2 oder 4 erkennen, und Formulieren dieses Polypeptids in einer Menge wirksam, um die Antikörper in einem Säugetier zu induzieren, mit einem pharmazeutisch annehmbaren Träger.

7. Vakzinzüsammensetzung zum Schutz eines Subjekts gegen Neisseria-Erkrankung umfassend in einem geeigneten Nukleinsäurezuführvehikel eine wirksame Menge einer Nukleinsäuresequenz ausgewählt aus der Gruppe bestehend aus:
(a) Seq. ID Nr. 1;
(b) Seq. ID Nr. 3;
(c) ein Fragment von Seq. ID Nr. 1 oder 3 bestehend aus 15 oder mehr aufeinanderfolgende Nukleinsäuren davon;
(d) eine Sequenz kodierend ein Polypeptid, das mindestens 90 % Identität mit entweder der Seq. ID Nr. 2 oder Seq. ID Nr. 4 aufweist und
(e) eine Sequenz von einem aus (a) bis (d) weiterhin kodierend ein zweites Polypeptid oder Protein.

8. Zusammensetzung nach Anspruch 7, wobei diese mehr als eine Nukleinsäure von (a) bis (e) umfasst.

9. Zusammensetzung nach Anspruch 7 oder 8, weiterhin umfassend mindestens eine andere Nukleinsäuresequenz kadlerend für ein Antigen oder ein Fragment davon aus einer heterologen oder homologen pathogenen Bakterienart.

10. Zusammensetzung nach Anspruch 9, wobei das andere Antigen mit einem von (a) bis (c) fusioniert ist.

11. Vakzinzusammensetzung zum Schutz eines Objekts gegenüber Neisseria-Erkrankung umfassend einen isolierten Antikörper, der ein Protein oder Peptid bindet, ausgewählt aus der Gruppe bestehend aus
(a) dem Protein gemäß Seq. ID Nr. 2;
(b) dem Protein gemäß Seq. ID Nr. 4;
(c) ein Peptidfragment von Seq. ID Nr. 2 oder 4 bestehend aus 8 oder mehr aufeinander folgenden Aminosäuren davon; und
(d) ein Protein oder Peptid, das mindestens 90 % Identität mit eine der Sequenzen von (a) bis (b) aufzeigt.

12. Zusammensetzung nach Anspruch 11 umfassend einen einzelnen isolierten Antikörper.

13. Zusammensetzung nach Anspruch 11 umfassend mehrere Antikörper.

14. Verwendung einer Zusammensetzung nach einem der Ansprüche 1 bis 13 zur Herstellung eines Medikaments zum Schutz eines Subjekts gegenüber Naisserien-Erkrankung.

## Revendications

1. Composition de vaccin pour la protection d'un sujet contre une maladie par *Neisseria* comprenant, dans un véhicule acceptable sur le plan pharmaceutique, une quantité efficace d'un polypeptide choisi dans le groupe constitué par :
(a) la protéine de SEQ ID N° 2 ;
(b) la protéine de SEQ ID N° 4 ;
(c) un fragment peptidique de SEQ ID N° 2 ou 4 composé de huit de leurs acides aminés consécutifs ou plus ;
(d) une protéine ou un peptide partageant au moins 90% d'identité avec l'une quelconque des séquences (a) et (b) ; et
(e) une séquence selon l'une quelconque des séquences (a) à (d) fusionnée à un deuxième polypeptide ou protéine.

2. Composition selon la revendication 1, qui comprend plus d'une protéine ou peptide selon l'une quelconque des séquences (a) à (e).

3. Composition selon la revendication 1 ou 2, comprenant en outre au moins un autre antigène ou un de ses fragments provenant d'une espèce bactérienne pathogène hétérologue ou homologue.

4. Composition selon la revendication 3, dans laquelle ledit autre antigène est fusionné à l'une des séquences (a) à (c).

5. Composition selon l'une quelconque des revendications précédentes, dans laquelle ledit polypeptide ne contient pas la séquence signal recouvrant les acides aminés 1 à 21 de SEQ ID N° 2 ou 4.

6. Procédé de fabrication d'une composition selon l'une quelconque des revendications précédentes, comprenant les étapes consistant à isoler un polypeptide recombiné comprenant au moins huit acides aminés consécutifs provenant de la séquence d'acides aminés de SEQ ID N° 2 ou 4, lequel polypeptide induit des anticorps qui reconnaissent la SEQ ID N° 2 ou 4, et à formuler ledit polypeptide en quantité efficace pour induire lesdits anticorps chez un sujet mammifère, avec un véhicule acceptable sur le plan pharmaceutique.

7. Composition de vaccin pour la protection d'un sujet contre une maladie par *Neisseria* comprenant, dans un véhicule approprié délivrant de l'acide nucléique, une quantité efficace d'une séquence d'acide nucléique choisie dans le groupe constitué par :
(a) SEQ ID N° 1;
(b) SEQ ID N° 3 ;
(c) un fragment de SEQ ID N° 1 ou 3 constitué de 15 de leurs acides nucléiques consécutifs ou plus ;
(d) une séquence codant un polypeptide partageant au moins 90% d'identité soit avec SEQ ID N° 2, soit avec SEQ ID N° 4 ; et
(e) une séquence selon l'une quelconque des séquences (a) à (d) codant en outre un deuxième polypeptide ou protéine.

8. Composition selon la revendication 7, qui comprend plus d'une séquence d'acide nucléique parmi les séquences (a) à (e).

9. Composition selon la revendication 7 ou 8, comprenant en outre au moins une autre séquence d'acide nucléique codant un antigène ou un de ses fragments provenant d'une espèce bactérienne pathogène hétérologue ou homologue.

10. Composition selon la revendication 9, dans laquelle ledit autre antigène est fusionné à l'une des séquences (a) à (c).

11. Composition de vaccin pour la protection d'un sujet contre une maladie par *Neisseria* comprenant un anticorps isolé qui se lie à une protéine ou à un peptide choisi dans le groupe constitué par :
(a) la protéine de SEQ ID N° 2 ;
(b) la protéine de SEQ ID N° 4 ;
(c) un fragment peptidique de SEQ ID N° 2 ou 4 constitué de huit de leurs acides aminés consécutifs ou plus ; et
(d) une protéine ou un peptide partageant au moins 90% d'identité avec l'une quelconque des séquences (a) et (b) ;

12. Composition selon la revendication 11, comprenant un seul anticorps isolé.

13. Composition selon la revendication 11, comprenant plusieurs anticorps.

14. Utilisation d'une composition selon l'une quelconque des revendications 1 à 13 dans la fabrication d'un médicament pour la protection d'un sujet contre une maladie par *Neisseria.*
